# EUROPÄISCHE PATENTSCHRIFT

(11) **EP 2 139 530 B1**
(45) Veröffentlichungstag und Bekanntmachung des Hinweises auf die Patenterteilung: **24.08.2011**
(21) Anmeldenummer: 08718273.9
(22) Anmeldetag: 27.03.2008
(51) Int. Cl.: A61L 24/00, A61L 24/04, A61L 27/44, A61L 27/14

(54) **IMPLANTATMATERIAL AUF BASIS EINES POLYMERSYSTEMS UND DESSEN VERWENDUNG**
IMPLANT MATERIAL BASED ON A POLYMER SYSTEM AND THE USE THEREOF
MATÉRIAU POUR IMPLANT À BASE D'UN SYSTÈME POLYMÈRE, ET SON UTILISATION

(30) Priorität: 27.03.2007 DE 102007015698
(43) Veröffentlichungstag der Anmeldung: 06.01.2010
(73) Patentinhaber: InnoTERE GmbH, 01307 Dresden (DE)
(72) Erfinder: NIES, Berthold, 64407 Fränkisch-Crumbach (DE)
(74) Vertreter: Kailuweit & Uhlemann Patentanwälte
(86) Internationale Anmeldenummer: PCT/EP2008/053640
(87) Internationale Veröffentlichungsnummer: WO 2008/116905

(56) Entgegenhaltungen:
- EP-A- 2 052 748
- WO-A-98/24398
- US-A- 5 797 873
- US-A1- 2008 039 586

## Beschreibung

Die Erfindung betrifft ein Implantatmaterial auf Basis eines Polymersystems aus mindestens 2 Komponenten und dessen Verwendung als Knochenzement, Knochenkleber, Gewebeersatzmaterial oder Wirkstoffträger.

Polymerbasierte Knochenzemente werden klinisch vor allem für die Befestigung von Gelenkimplantaten eingesetzt. Sie sind seit ca. 50 Jahren in der klinischen Praxis etabliert und werden heute weltweit in ca. 5 Mio. Fällen jährlich verwendet. Die chemische Zusammensetzung der Knochenzemente ist in dieser Zeit praktisch unverändert geblieben. Sie besteht im wesentlichen aus einer Pulverkomponente, die ein oder mehrere Polymere vor allem bestehend aus Acrylaten, Methacrylaten und Styrol, bzw. Copolymere aus diesen Monomeren oder Mischungen aus den entsprechenden Homo- und/oder Copolymeren enthalten (zusammenfassend als **PMMA** bezeichnet). Weitere Bestandteile der Pulverkomponente sind in der Regel ein Röntgenkontrastmittel und ein Radikalstarter. Als Röntgenkontrastmittel kommen vorzugsweise Bariumsulfat oder Zirkondioxyd zum Einsatz. Als Radikalstarter wird in allen kommerziellen Knochenzementen di-Benzoylperoxid **(BPO)** eingesetzt. Die zweite Knochenzementkomponente ist eine reaktive organische Flüssigkeit, die ganz überwiegend aus dem Monomer Methyl-Methacrylat **(MMA)** besteht und in seltenen Fällen auch andere Ester der Acrylsäure oder Methacrylsäure enthält. Weitere Bestandteile sind ein Co-Starter (auch als Aktivator oder Co-Initiator bezeichnet) und ein Stabilisator oder Inhibitor. Als Co-Starter wird in fast allen kommerziellen Knochenzementen Dimethyl-p-Toluidin **(DMPT)** verwendet, sehr selten ein anderes tertiäres Amin. Als Inhibitor wird vorwiegend Hydrochinon oder eines seiner Derivate verwendet.

Zusätzlich können Knochenzemente noch weitere Substanzen enthalten (Antibiotika, Farbstoffe), die bei der vorliegenden Betrachtung aber zunächst unberücksichtigt bleiben können.

Werden bei einem konventionellen Knochenzement Pulver und Flüssigkeit miteinander gemischt, so reagieren Initiator (BPO) und Co-Starter (DMPT) miteinander unter Bildung von Radikalen, die wiederum an den Doppelbindungen der Monomer-Moleküle angreifen und eine Polymerisations-(Ketten)-Reaktion auslösen, solange, bis der überwiegende Teil des Monomers zu Polymerketten abreagiert ist. Parallel löst das Monomer einen Teil des Polymers an bzw. auf, was initial zu einer schnellen Erhöhung der Viskosität der Zementmasse führen kann und wodurch eine innige Verbindung von Pulver und auspolymerisierender Flüssigkeit erreicht wird. Die komplette Abbindereaktion von der Mischung bis zur vollen Belastbarkeit ist bei den konventionellen PMMA-Knochenzementen in ca. 10 - 30 Minuten abgeschlossen. Trotz der langen Erfahrung und des verbreiteten Gebrauchs haben PMMA-Knochenzemente eine Reihe von Nachteilen:
■ Anmischung: Das Zementpulver ist eine Mischung sehr feiner Pulver, die ihrerseits sehr unterschiedliche Eigenschaften haben (Partikelgröße, Dichteunterschiede von 1,18 für PMMA und 5,85 für ZrO₂) und daher nur sehr schwer homogen gemischt werden können und eines entsprechenden Herstellaufwandes bedürfen. Die Mischung des Zementpulvers mit der Monomerflüssigkeit ist ebenfalls problematisch, weil die Viskosität der Flüssigkeit sehr schnell ansteigt und dann eine homogene Vermischung erschwert. Eine weitgehend porenfreie Zementmasse wird praktisch nur bei Verwendung von aufwendigen und teuren Mischsystemen erreicht.
■ Schwindung: Während der Polymerisationsreaktion nimmt die Dichte beim Übergang von reinem Monomer zu Polymer um mehr als 20% zu, entsprechend nimmt das Volumen ab. Da der Knochenzement großenteils bereits auspolymerisiertes Material enthält (PMMA-Anteil), liegt in diesem System die Schwindung deutlich niedriger und wird mit ca. 2-5% angegeben (Kühn, Bone Cements, Springer Verlag, 2000, ISBN 3-540-67207-9). Neben der hohen Polymerisationswärme kann die Schwindung als ein wesentlicher klinisch relevanter Nachteil der konventionellen Knochenzemente angesehen werden, der in wichtigen klinischen Indikationen die Einsatzmöglichkeiten begrenzt. Bei großen notwendigen Schichtdicken (wie beispielsweise bei Prothesenwechseln) kann die Schwindung dazu führen, dass sich ein ausgeprägter Spalt zwischen Zement und Knochen bildet und eine physiologische Kraftübertragung nicht mehr möglich ist.
■ Polymerisationswärme: Die Polymerisationsreaktion von MMA zu PMMA ist stark exotherm. Die erzielten Spitzentemperaturen nach ISO 5833 liegen bei ca. 80°C und hängen ganz wesentlich vom Mengenverhältnis von Monomer zur gesamten Zementmasse und zu einem geringeren Anteil von der Polymerisationskinetik ab. Klinisch relevant ist die hohe Polymerisationswärme besonders bei großen Mengen einzubringenden Zements, wenn das umliegende Gewebe die anfallende Wärme nicht schnell genug abführen kann, um Gewebsnekrosen zu vermeiden.
■ Mechanik: Für die meisten der heute üblichen Anwendungen hat PMMA-Knochenzement ausreichende mechanische Eigenschaften. Für einige neue Anwendungen - insbesondere die Vertebroplastie oder generell die Versteifung von spongiösem Knochen - wird die hohe Steifigkeit aber vielfach als Nachteil angesehen. Eine reduzierte Steifigkeit könnte auch in vielen anderen - auch den traditionellen - Anwendungsgebieten klinische Vorteile bringen.
■ Wirkstofffreisetzung: Die Mehrzahl der Knochenzementanwendungen entfällt heute in vielen Ländern auf die Antibiotika-haltigen Versionen zur Prophylaxe von Fremdkörper-assoziierten Infektionen. Um ausreichende Freisetzungen zu erreichen, müssen sehr hohe Dosen in den Zement eingemischt werden, wovon der überwiegende Teil über sehr lange Zeiträume in sehr niedrigen Konzentrationen (oder gar nicht) freigegeben wird. Diese Tatsache wird vielfach mit der Entwicklung und Ausbreitung resistenter Bakterienstämme in Verbindung gebracht. Die Erzielung ausreichender Wirkspiegel bei sehr viel niedrigeren Dosen, die über einen kürzeren Zeitraum vollständig freigesetzt werden können, ist daher anzustreben.
■ Gewebeverträglichkeit: PMMA ist ausreichend gewebeverträglich und erfüllt die standardisierten Anforderungen an die Biokompatibilität von Implantatmaterialien. PMMA wird allerdings nicht knöchern integriert, sondern durch Narbengewege eingekapselt. Das hat sowohl biochemische Ursachen, als auch strukturelle Gründe. Solider Knochenzement bietet dem umgebenden Knochen keine Möglichkeit in ein äußeres Porensystem einzuwachsen und damit eine sekundäre Verzahnung zu erreichen, so wie dies bei modernen unzementierten permanenten Metallimplantaten der Fall ist.

Aus WO 2005/009481 A1 ist ein tensidhaltiger Knochenzement aus einer flüssigen und festen Komponente bekannt. Die Pulverkomponente ist gegenüber konventionellen Knochenzementen nicht verändert, lediglich die flüssige Komponente enthält neben dem Monomer einen Tensid und einen Beschleuniger. Erst vor Einsatz werden die Komponenten gemischt. Ziel von WO 2005/009481 A1 ist es, einem herkömmlichen Knochenzement eine verbesserte Freisetzung von Antibiotika-Wirkstoffen zu verleihen.

WO 2004/071543 A1 offenbart eine injizierbare Knochenersatz-Mischung aus a) einem Zweikomponenten-Pulverflüssigkeitsknochenzement, b) einer weiteren Komponente, die nicht mit der Zementpaste mischbar ist, und c) einem Röntgenkontrastmittel. Nach Mischung der Komponenten soll sich ein selbsthärtendes, poröses Knochenersatzmaterial bilden, bei der Komponente b nach Aushärtung ausgewaschen werden kann. Die Lehre nach WO 2004/071543 A1 beschränkt sich auf ein konventionelles Pulverflüssigkeitssystem, dem erst nach der Vermischung der konventionellen Komponenten eine nicht mischbare Flüssigkeit zur Porenbildung zugesetzt wird.

DE 3245956 A1 betrifft ein chirurgisches Material auf der Basis von flüssigen, monomeren und pulverförmigen polymeren Acryl- und/oder Methacrylsäureestern, Katalysatoren, Beschleunigern und gegebenenfalls Zuschlagstoffen, bei dem die flüssige Komponente keine wässrige Emulsion sondern eine Lösung mit speziellen organischen Flüssigkeiten ist, die an der Polymerisationsreaktion nicht teilnehmen und zu einer verminderten Temperaturentwicklung bei Mischung und Einbau des chirurgischen Materials führen.

US 4,093,576 offenbart eine Knochenzementmischung aus einem Polymerpulver und einem damit kompatiblem hochviskosen wasserlöslichen Gel von mehr als 200 000 centipoise. Nach Mischung dieser Komponenten entsteht ein poröses Knochenersatzmaterial.

Gegenstand von DE 10 2004 049 A1 ist ein Antibiotikum-/Antibiotika enthaltender PMMA-Knochenzement mit einer Pulverkomponente und einer flüssigen Komponente. Dabei soll aufgrund der spezifischen Zusammensetzung der zugesetzten Antibiotika, deren Freisetzung signifikant erhöht werden.

Zementartige Zusammensetzungen, die aus zwei Pasten bestehen und in Doppelkammerspritzen angeboten und über Statikmischer kombiniert und zur Reaktion gebracht werden, sind vor allem aus der zahnärztlichen Praxis seit längerer Zeit bekannt. Ein davon abgeleitetes Produkt (Cortoss der Firma Orthovita) ist in den vergangenen Jahren auch für das Gebiet der Orthopädie entwickelt worden. Sowohl die zahnärztlichen Füllungsmaterialien als auch das Produkt Cortoss unterscheiden sich aufgrund ihres Anteiles an glaskeramischen Füllmaterialien in der ersten Paste deutlich von den konventionellen Knochenzementen. Als Startersystem wird BPO/DMPT verwendet, wobei DMPT in der ersten Paste enthalten ist und BPO in der zweiten Paste gelöst vorliegt und nur unter Kühlung lagerstabil ist. Eine ausreichende Lagerstabilität ist bei diesem Produkt nur bei kontinuierlicher Kühlung gewährleistet und die in hoher Konzentration zugesetzten mineralischen Füllstoffe neigen trotz der hohen Viskositäten zur Sedimentation. Von Produkten wie Cortoss und zahnärztlichen Füllungsmaterialien grenzt sich das erfindungsgemäße Implantatmaterial deutlich ab, indem die erfindungsgemäßen Materialien immer eine Suspension von Polymerpulvern in Trägerflüssigkeiten enthalten, in denen sie weder löslich noch signifikant quellbar sind. Weiterhin gibt es eine deutliche stoffliche Abgrenzung, indem Produkte wie Cortoss als Monomere in beiden Zementkomponenten vorwiegend Makromere mit mehr als einer Doppelbindung enthalten, während die Monomerflüssigkeit in den erfindungsgemäßen Materialien überwiegend aus dem einwertigen MMA und darin gelösten Polymeren besteht.

In der wissenschaftlichen Literatur sind in den vergangenen Jahren auch Veröffentlichungen zu 2-Pasten-PMMA-Zementen erschienen, die auf konventionellen Knochenzementen basieren (Li et al., Bioactive and osteoporotic bone cement, US 6,593,394 B1; Gilbert JL, Hasenwinkel JM, Wixson RL, Lautenschlager EP; J Biomed Mater Res. 2000 Oct;52(1):210-8). In diesen Fällen handelt es sich ausschließlich um hochviskose Lösungen von PMMA-Copolymeren in MMA mit hohen Gehalten an mineralischen Füllmaterialien, bei denen die eine Paste das BPO und die andere das DMPT enthält. Diese Zusammensetzungen haben damit die gleichen technologischen Nachteile wie Cortoss in bezug auf Lagerstabilität und Sedimentation. Nachteilig ist hier ebenfalls die sehr hohe Wärmeentwicklung bei der Polymerisation, die durch den hohen notwendigen MMA-Gehalt für die Pastenpräparation bedingt ist.

In den frühen 80er Jahren des 20. Jahrhunderts wurde von der Firma Beiersdorf ein Knochenzement entwickelt und am Markt eingeführt, der in der Pulverkomponente eine konventionelle Zusammensetzung hatte, aber als Monomerflüssigkeit eine Emulsion aus ca. 10% Wasser in MMA aufwies. Ziel war vor allem die Absenkung der Polymerisationstemperatur. Bis auf die Verwendung von Emulgatoren und wässrigen Komponenten in der Gesamtrezeptur bestehen keine prinzipiellen Gemeinsamkeiten mit der erfindungsgemäßen Zusammensetzung.

Von klinischen Fragestellungen in Verbindung mit Anforderungen an Knochenzementen für die Vertebroplastie inspiriert, wurden in den Vergangenen Jahren eine Reihe von Untersuchungen zur Abmischung von Knochenzementen mit wässrigen Polymerlösungen, insbesondere Hyaluronsäure, durchgeführt, um die Steifigkeit der Zemente herabzusetzen. Nach Boger A., Verrier S., Bohner M., Heini P., Schneider E., - Injizierbarer poröser Knochenzement für die Vertebroplastik mit physiologisch angepassten mechanischen Eigenschaften, Bern; DGU, 2005 - wurden zunächst konventionelle Knochenzemente angemischt und anschließend mit Hyaluronsäure vermischt. Diese Vorgehensweise führt anders als beim erfindungsgemäßen Vorgehen zu wenig reproduzierbaren Ergebnissen und schon bei relativ niedrigen Mengen an Hyaluronsäure zu dramatischen Festigkeitsverlusten. Der wesentliche Grund für die unbefriedigenden Ergebnisse ist die unter OP-Bedingungen und mit Mitteln, wie sie dort zur Verfügung stehen, praktisch nicht erreichbare gleichmäßige Dispergierung von wässrigen Lösungen in einer bereits angemischten Knochenzementpaste. Entsprechend inhomogen sind die erhaltenen Zementmassen, so dass diese Methode für den klinischen Einsatz nicht praktikabel ist. Die zitierten Arbeiten sind daher in keiner Weise eine Vorwegnahme der aktuellen Erfindung, da weder die konkrete Lehre beschrieben wird, noch die erzielten Ergebnisse erreicht werden.

Insgesamt zeigen die genannten Arbeiten, dass ein reges Interesse an einer Verbesserung von konventionellen Knochenzementen besteht, dass die bisher beschrittenen Lösungswege aber noch weit von einer befriedigenden Lösung entfernt sind.

Die vorliegende Erfindung knüpft an den Schwachstellen konventioneller PMMA-Knochenzemente an, indem sie einen neuen Ansatz zur Präparation und stofflichen Zusammensetzung von Knochenzementen verfolgt, während sie aber dennoch auf den etablierten Ausgangsmaterialien aufbaut. Auf diesem Wege sollen Implantatmaterialien, insbesondere für Knochenzemente, für die Vertebroplastie und die Auffüllung von Knochendefekten im Rahmen von Prothesenrevisionen und für die Augmentierung von osteoporotischem Knochen geschaffen aber auch Materialien für nichtmedizinische Anwendungsgebiete erschlossen werden.

Erfindungsgemäß wird die Aufgabe durch ein Implantatmaterial mit den Merkmalen nach Anspruch 1 gelöst. Ausgestaltungen und Anwendungen dieses Implantationsmaterials beinhalten die Ansprüche 2 bis 14.

Das erfindungsgemäße Implantatmaterial auf Basis eines bioverträglichen Polymersystems ist aus mindestens 2 Komponenten, die bei Vermischung miteinander reagieren und einen polymerbasierten Feststoff bilden, zusammengesetzt, wobei mindestens die erste Komponente des Polymersystems eine Lagerstabile Paste aus mindestens einem bioverträglichen Polymerpulver und einer Starterkomponente bzw. einem Starter zur Auslösung einer Polymerisationsreaktion bei Vermischung ist.

Erfindungsgemäß ist die erste Komponente des Polymersystems eine lagerstabile Paste aus mindestens einem bioverträglichen Polymerpulver und einer Starterkomponente bzw. einem Starter und einer Trägerflüssigkeit, wobei die Trägerflüssigkeit so ausgewählt ist, dass sich unter Normalbedingungen das Polymerpulver nicht auflöst oder signifikant quillt und die Starterkomponente bis zur Vermischung der Komponenten des Polymersystems stabil bleibt.

Die zweite Komponente des Polymersystems enthält mindestens eine reaktive organische Flüssigkeit oder eine Lösung oder eine Suspension einer reaktiven organischen Flüssigkeit und eines bioverträglichen Polymers.

Erfindungsgemäß ist die erste Komponente des Polymersystems als Paste ausgeführt, wobei sich die Zusammensetzung dieser Paste vorzugsweise an die Zusammensetzung der Pulverkomponente konventioneller Knochenzemente anlehnt, d. h. Polymerpulver, und Radikalstarter (bevorzugt BPO) und optional Röntgenkontrastmittel und ggf. Wirkstoffe enthält. Diese Komponenten werden mit der Trägerflüssigkeit so kombiniert, dass sich das Polymerpulver und die Starterkomponente nicht in der Trägerflüssigkeit auflösen, sondern suspendiert werden. Die Suspension liegt in der erfindungsgemäßen Form als Paste vor. Definitionsgemäß sind Pasten Suspensionen von Feststoffen in Flüssigkeiten mit einem hohen Feststoffgehalt. Pasten sind üblicherweise nicht fließfähig aber leicht verformbar. Im Rahmen dieser Erfindung soll der Pastenbegriff in anschaulicher Form verwendet werden, da eine exakte Eingrenzung über Viskositäten nicht vorliegt. Als Pasten werden danach halbfeste Massen angesehen, die in etwa die Bereiche abdecken, die auch für Zahnpasta üblich sind. Der Pastenbegriff soll allerdings ausschließlich dazu dienen, die erste Komponente des Polymersystems von pulvrigen, granulären oder sonstigen festen Massen abzugrenzen.

Kernpunkt der Erfindung ist die neuartige Formulierung der Pulverkomponente von weitgehend aus konventionellen Materialien zusammengesetzten Knochenzementen, wie sie z. B. in Kühn, Bone Cements, Springer Verlag 2000 beschrieben sind. Hierzu werden die üblichen Pulverbestandteile - Polymerpulver, Röntgenkontrastmittel (sofern enthalten) und Starterkomponente und ggf. weitere Zuätze als eine Paste oder Suspension in einer Trägerflüssigkeit formuliert, in der diese Bestandteile (insbesondere die Starterkomponente) unter Normalbedingungen stabil sind. Normalbedingungen sind definiert als 25°C und 101,3 kPa.

Das bioverträgliche Polymerpulver der ersten Komponente des Polymersystems ist ausgewählt aus Homo- oder Co-Polymerisaten von Acryl-, Methacrylsäureestern, Styrol-, Vinyl-Derivaten oder deren Mischungen.

Das erfindungsgemäße Implantatmaterial auf Basis eines selbst-/kalthärtenden Polymersystem baut in seiner bevorzugten Ausführungsform auf handelsüblichen Knochenzementen und deren Produktanforderungen auf, um an den langjährigen Erfahrungen mit diesen Materialien anknüpfen zu können. Einen umfassenden Überblick über diese Produktgruppe gibt die Monographie Bone Cements (Kühn, Springer Verlag, 2000, ISBN 3-540-67207-9). Die erfindungsgemäße Technologie eröffnet erstmals die Möglichkeit, in der Zusammensetzung und im Eigenschaftsspektrum weit über die Grenzen bisheriger Knochenzemente hinauszugehen, die sich durch Pulver/Flüssigkeits-Systeme erreichen lassen.

Die Auswahl der geeigneten Polymere ergibt sich ausschließlich aus ihrer Verträglichkeit mit den erfindungsgemäß einzusetzenden Monomersystemen. Die Verträglichkeit von Polymeren untereinander wird durch ihre chemische Struktur sehr stark beeinflusst und begrenzt. Diese Tatsache behindert einerseits die Auswahlmöglichkeiten bei der Entwicklung und Herstellung von Polymerblends, wird andererseits aber auch gezielt genutzt, um durch Entmischungsvorgänge und gezielten Einbau von Inhomogenitäten die mechanischen Eigenschaften, das Bruchverhalten, tribologische Eigenschaften oder sonstige Oberflächeneigenschaften zu beeinflussen. Auch wenn in der vorliegenden Erfindung im Hinblick auf die bevorzugte Verwendung der Materialien als Knochenzemente Polymersysteme auf der Basis von Homo- oder Co-Polymerisaten von Acryl- und/oder Methacrylsäureestern, Styrol-, Vinyl-Derivaten und/oder deren Mischungen im Vordergrund stehen, sind hier doch ausdrücklich alle Polymere geeignet und beansprucht, die sich in polymerisierbaren Monomeren und Makromeren an- oder auflösen lassen oder, die sich in einer Matrix aus polymerisierbaren Monomeren oder Monomerlösungen fest einbinden lassen.

Zur Herstellung der ersten Komponente als Paste werden mindesten ein Polymer (Pulver) und ein Starter (der bereits im Polymer enthalten sein kann) in einer Trägerflüssigkeit suspendiert. Die Trägerflüssigkeit ist vorzugsweise Wasser oder eine wässrige Lösung (wie oben ausgeführt kommen prinzipiell aber auch alle anderen Flüssigkeiten in Betracht, die weder für den Starter, noch für das Polymer als Lösungsmittel dienen). Im Hinblick auf die bevorzugte Verwendung als Knochenzement sind bioverträgliche und als pharmazeutische Hilfsstoffe etablierte Flüssigkeiten besonders bevorzugt, insbesondere neben Wasser und wässrigen Lösungen, Glycerin, Glycerinester, Propandiol, niedermolekulare PEG, PEG-PPG-Copolymere, DMSO, Methyl-Pyrrolidon, bioverträgliche Öle, deren Mischungen untereinander und mit anderen Substanzen.

Als Starterkomponente bzw. Starter wird vorzugsweise ein Peroxid, bevorzugt BPO verwendet. Die Verwendung von BPO in konventionellen Knochenzementen ist seit Jahrzehnten etabliert. In diesen Fällen liegt das BPO in der Pulverkomponente vor und ist mit Wasser phlegmatisiert (in besonderen Fällen liegt das BPO einpolymerisiert in der Polymerkomponente vor, was ebenfalls eine Phlegmatisierung bewirkt). In phlegmatisierter Form ist das BPO über einen ausgedehnten Zeitraum lagerstabil - handelsübliche Knochenzemente weisen eine Lagerstabilität von bis zu 5 Jahren aus.

In handelsüblichen 2-Komponenten-Pastensystemen (Cortoss oder dentale Füllmaterialien) liegt das BPO in einer der Komponenten in gelöster Form vor. In dieser Form ist das BPO nicht phlegmatisiert und zerfällt in Abhängigkeit von der Temperatur spontan. Die Stabilität des BPO begrenzt daher die Lagerfähigkeit dieses Produkts. Zur Erhöhung der Lagerfähigkeit müssen deshalb entsprechende Produkte gekühlt werden, was aus Logistikgründen unerwünscht ist.

Beim erfindungsgemäßen Implantatmaterial liegt die Starterkomponente in der Trägerflüssigkeit in ungelöster Form vor. Bei Verwendung von Peroxid, ist das Peroxid bevorzugt BPO wie in konventionellen Knochenzementen, vorzugsweise mit Wasser phlegmatisiert. Es gibt daher keine nachteiligen Effekte für die Lagerstabilität.

Als Trägerflüssigkeit kommen alle bioverträglichen Flüssigkeiten in Betracht, in denen die Starterkomponente stabil ist und in denen sich das Polymerpulver nicht auflöst oder nicht signifikant (< 5%) quillt. Besonders bevorzugt sind Wasser und wässrige Lösungen, Glycerin, Glycerinester, Propandiol, niedermolekulare PEG, PEG-PPG-Copolymere, DMSO, Methyl-Pyrrolidon, bioverträgliche Öle, deren Mischungen untereinander und mit anderen Substanzen. Der Trägerflüssigkeit können verschiedene Substanzen zugemischt sein, die die Funktion haben, einerseits die Pulverbestandteile effektiv in der Flüssigkeit zu suspendieren und andererseits die biologischen, mechanischen und strukturellen Eigenschaften des Knochenzements zu beeinflussen.

Die zweite Komponente des Polymersystems enthält mindestens eine reaktive organische Flüssigkeit oder eine Lösung oder eine Suspension einer reaktiven organischen Flüssigkeit und eines Polymers. Die reaktive organische Flüssigkeit ist dabei aus Methylmethacrylat oder homologen Estern der Methacrylsäure oder deren Mischungen ausgewählt.

Die zweite Komponente des erfindungsgemäßen Polymersystems ist gegenüber konventionellen Knochenzementen weniger stark verändert. Modifikationen beziehen sich hier im wesentlichen auf Maßnahmen zur Beeinflussung der Viskosität und des Benetzungsverhaltens, so dass beide Komponenten aufeinander abgestimmt werden können und sich leicht in der gewünschten Weise miteinander mischen lassen.

Das Ziel dieser Abstimmung kann einerseits darin bestehen, dass beide Komponenten homogen miteinander vermischt werden können und andererseits darin, dass bei der Vermischung vorbedachte und kontrollierte Inhomogenitäten auftreten können. Im ersten Fall lassen sich die - modifizierte - Monomerflüssigkeit und die Trägerflüssigkeit der Paste bzw. der Pulversuspension miteinander mischen, so dass eine molekulare Verteilung ohne die Ausbildung von Phasengrenzen zwischen den verwendeten Flüssigkeiten entsteht. In diesem Fall entspricht die Art und Weise der Polymerisation einer Lösungspolymerisation, bei der das Lösungsmittel nach dem Abschluss der Polymerisation entweder permanent in der Zementmasse verbleibt oder später teilweise oder vollständig in das umgebende Medium freigesetzt werden kann.

Im zweiten und bevorzugten Fall bilden erste und zweite Komponente des Polymersystems (Paste bzw. Pulversuspension und - modifizierte - Monomerflüssigkeit) bei der Vermischung eine physikalische Mischung im Sinne einer Emulsion, in der die Trägerflüssigkeit der Paste und die Monomerflüssigkeit getrennte Phasen ausbilden. Diese physikalischen Mischungen werden durch die Verwendung geeigneter Emulgatoren/Tenside bzw. deren Mischungen begünstigt und falls nötig durch Stabilisatoren unterstützt und zumindest für die Dauer der Vermischung bis zum Abschluss der Polymerisationsreaktion stabilisiert. In diesem Fall entspricht die Art und Weise der Polymerisation einer Suspensions- oder Emulsionspolymerisation, bei der nach der innigen Vermischung der beiden Komponenten dic - modifizierte - Monomerflüssigkeit und Bestandteile der Pulverpaste die kontinuierliche Phase bilden, während die suspendierte Phase im wesentlichen aus der Trägerflüssigkeit der ursprünglichen Pulverpaste gebildet wird. Die eigentliche Polymerisation findet in diesem Fall praktisch ausschließlich in der kontinuierlichen Phase statt. Beide Komponenten können so aufeinander abgestimmt werden, dass die suspendierte Phase isolierte Tröpfchen oder flüssigkeitsgefüllte Poren in der auspolymerisierten kontinuierlichen Phase bildet oder dass die suspendierte Phase ein interpenetrierendes Netzwerk mit der kontinuierlichen Phase, also ein weitgehend interkonnektierendes Porensystem mit Verbindung zum äußeren Medium ausbildet.

Versuche haben überraschend gezeigt, dass sich aus der Pulverkomponente konventioneller Knochenzemente (z. B. Palacos® der Firma Biomet-Merck, bzw. Heraeus Medical), einer wässrigen Polymerlösung (z. B. Carboxymethylstärke) und geeigneter bioverträglicher Tenside (z. B. Tween® 80) lagerstabile Pasten herstellen lassen. Diese Pasten lassen sich in ausgezeichneter Weise mit einer konventionellen Monomerlösung (z. B. MMA-Lösung für Palacos® der Firma Biomet-Merck, bzw Heraeus Medical), in der geringe Mengen PMMAPMA-Copolymer (z. B. Degacryl®, der Firma Degussa) gelöst sind, vermischen, wobei nach vollständiger Vermischung innerhalb kurzer Zeit eine makroskopisch homogene Emulsion gebildet wird, die ebenfalls in kurzer Zeit (< 10 min) spontan polymerisiert und eine feste poröse Zementmasse ausbildet.

Die vorbedachte Auswahl, ob sich im Zuge der Abbindereaktion eine solide oder poröse Struktur ausbildet, lässt sich insbesondere durch die Auswahl der Trägerflüssigkeit der ersten Komponente des Polymersystems erreichen. Wenn die Trägerflüssigkeit der ersten Komponente mit der Monomerflüssigkeit der 2. Komponente des Polymersystems mischbar ist, erhält man vorwiegend einen soliden Feststoff, während die Verwendung einer Trägerflüssigkeit, die nicht mit der Monomerflüssigkeit mischbar ist, einen porösen Feststoff ergibt. Bei der Unterscheidung beider Fälle ist der Begriff der Mischbarkeit von Bedeutung. Bei vollständiger Mischbarkeit oder chemischen Mischungen liegen die gemischten Substanzen molekular verteilt vor, d. h. die Mischungen sind bis in den molekularen Bereich homogen und es liegen keine Phasengrenzen zwischen den gemischten Substanzen vor; Beispiele sind Lösungen von Substanzen ineinander. Im vorliegenden Fall trifft dies auf Propandiol als Trägerflüssigkeit zu, das sich mit dem MMA-Monomer mischen lässt. Die Polymerisation ergibt dann einen Feststoff, der nahezu porenfrei ist. Das eingebrachte Propandiol liegt nach der Polymerisation (molekular) verteilt in der Polymermatrix vor.

Liegt keine oder keine vollständige Mischbarkeit der Substanzen vor, bilden sich Phasengrenzen zwischen den Substanzen aus, indem sich Suspensionen (fest in flüssig) oder Emulsionen (flüssig in flüssig) bilden können. Im vorliegenden Fall trifft das zu; wenn die Trägerflüssigkeit der ersten Komponente des Poymersystems und Monomerflüssigkeit nicht vollständig miteinander mischbar sind. Der bevorzugte Fall ist die Verwendung einer wässrigen Lösung als Trägerflüssigkeit der ersten Komponente des Polymersystems und einer PMMA-Copolymerlösung in MMA als zweite Komponente des Polymersystems. MMA ist nur geringfügig in Wasser löslich und daher kann sich bei Vermischung der beiden Komponenten keine homogene chemische Mischung ausbilden. Die Oberflächenspannung der beiden Flüssigkeiten und deren Polaritätsunterschiede bewirken eine Entmischung, die aber durch die Verwendung geeigneter oberflächenaktiver Substanzen (Tenside) gesteuert werden kann. Die Vermischung der beiden Komponenten des Polymersystems hat in diesem Fall zur Folge, dass sich die suspendierten Polymerpulver aus der ersten Komponente - wegen der ähnlichen Polarität - mit der MMA-Flüssigkeit kombinieren und das BPO (ebenfalls suspendiert in der ersten Komponente) wegen seiner guten Löslichkeit in MMA ebenfalls in das MMA übertritt. In der MMA-Phase kann dann die Polymerisation erfolgen, da nun BPO und DMPT beide in der gleichen Phase vorliegen und miteinander reagieren können. Das Vorhandensein der Polymerpulver und von Tensiden verbunden mit der erhöhten Viskosiät beider Komponenten verhindert ein vollständiges Zusammenfließen der beiden Phasen, sondern führt zur Ausbildung eines zusammenhängenden Porensystems, in dem die wässrige Lösung der Trägerflüssigkeit das Porensystem ausfüllt.

Überraschend sind in diesem Zusammenhang insbesondere folgende Punkte:
■ Die Vermischung von konventionellem Knochenzementpulver, einer wässrigen Polymerlösung und bioverträglichen Tensiden ergibt eine lagerstabile Paste mit makroskopischer Homogenität und ausgezeichneten Handhabungeigenschaften, z. B. können sehr hohe Feststoffgehalte eingestellt werden bei gleichzeitigem Erhalt der Extrudierbarkeit durch eine handelsübliche Spritze. Ein Absetzten von Bestandteilen der Pulverkomponente, insbesondere des Zirkondioxyds war nicht festzustellen.
■ Die Paste lässt sich mit einem in der Viskosität angepassten konventionellen Monomer leicht in einem einfachen Mischbecher oder einer an sich bekannten Doppelkammerspritze (im vorliegenden Fall der Firma Mixpac®, Schweiz) mischen, so dass sich eine makroskopisch homogene Zementmasse ergibt. Auch ohne besondere Maßnahmen werden bei offener Anmischung keine Makroporen in die Zementmasse eingebracht, so wie dies bei Pulver/Flüssigkeits-Mischungen regelmäßig der Fall ist.
■ In der erfindungsgemäßen Abmischung erfolgt die Polymerisationsreaktion der auf der Rezeptur des konventionellen Knochenzements (Palacos®) basierenden Rezeptur signifikant schneller als bei der vergleichbaren Rezeptur in Pulver/FlüssigkeitsMischungen.
■ Besonders überraschend ist das breite Spektrum von Mischungsverhältnissen zwischen beiden Komponenten der erfindungsgemäßen Rezepturen, innerhalb dessen Zementreaktionen mit vielversprechenden Eigenschaften ablaufen. Insbesondere können die Pulverpasten mit sehr viel geringeren Mengen an Monomerflüssigkeit angemischt werden, als dies bei Pulver/Flüssigkeits-Mischungen erreicht werden kann.
■ Die Exothermie lässt sich in Abhängigkeit von der Monomermenge signifikant reduzieren.
■ Trotz der mikroporösen Strukturen des ausgehärteten Zements lassen sich überraschend hohe Festigkeiten erreichen.
■ Die Mikroporen bilden je nach Einstellung der erfindungsgemäßen Zementrezeptur ein interkonnektierendes Porensystem aus. Diese Eigenschaft konnte bisher mit keinem selbsthärtenden Implantatmaterial, das eine nennenswerte strukturelle Festigkeit aufweist, erreicht werden.

Charakteristisch für die vorliegende Erfindung ist die Realisierung eines aus mindestens 2-Komponenten bestehenden selbsthärtenden Polymersystems, wobei eine erste Komponente eine Dispersion oder Suspension von Polymer in einer Trägerflüssigkeit enthält und diese Trägerflüssigkeit weder das Polymer lösen kann noch eine signifikante Quellung des Polymers in der Trägerflüssigkeit erfolgt. Damit kommen als Trägerflüssigkeiten praktisch alle Flüssigkeiten in Betracht, in denen Polymere, die für die Herstellung selbsthärtender Kunststoffsysteme geeignet sind, stabil sind. Weiteres Charakteristikum und Voraussetzung ist, dass auch das Startersystem mit der Trägerflüssigkeit kompatibel ist. In diesem Punkt unterscheidet sich die Erfindung grundlegend von bekannten 2-Pasten-Systemen, die durchweg entweder mehrwertige vernetzbare Makromere oder Lösungen von Polymeren in niedermolekularen, meist einwertigen Monomeren enthalten. Starter und Co-Starter bzw. Initiator liegen jeweils gelöst in einer der getrennten Pasten vor und werden bei der Vermischung der Pasten zur Reaktion gebracht, wodurch die Polymerisation ausgelöst wird. In der Regel enthalten diese 2-Pasten-Systeme noch verschiedene nicht reaktive Füllstoffe.

Wesentliche Bestandteile der Trägerflüssigkeit sind Substanzen, die eine Dispergierbarkeit des Polymers verbessern. Von großer Bedeutung sind weiterhin Substanzen, die zu einer Einstellung der Viskosität der Trägerflüssigkeit genutzt werden können. Hierzu zählen insbesondere Polymere, die in der Trägerflüssigkeit löslich sind und/oder viskose Flüssigkeiten, die mit der Trägerflüssigkeit mischbar sind. Des weiteren zählen Substanzen dazu, die als sehr feinteilige Dispersionen die Viskosität und Rheologie von Flüssigkeiten modifizieren können, wie insbesondere hochdisperse Silikate und Phosphate. Besonders bevorzugte Polymere zu Einstellung der Viskosität oder allgemeiner der Konsistenz der ersten Paste sind bioverträgliche wasserlösliche Polymere wie lösliche Stärke und Stärkederivate, Cellulosederivate, Kollagen, Gelatine, PEG oder PEO (Poly-Ethylenoxid), PEG-PPG-Copolymere, wasserlösliche modifizierte Polyacrylate/Polymethacrylate, PVP, PVA, etc.

Insbesondere bei wasserbasierten Polymerpasten ist der Zusatz von geeigneten oberflächenaktiven Substanzen vorteilhaft.

Weitere Zusätze zur ersten Komponente hängen vom vorgesehenen Einsatzgebiet ab und umfassen insbesondere die für Knochenzemente beschriebenen Röntgenkontrastmittel, Antibiotika, sonstige Wirkstoffe, Farbstoffe und Füllmaterialien.

Die zweite Komponente besteht in einem einfachen Fall aus dem (Knochenzement)-Monomer als reaktive organische Flüssigkeit, einem Co-Starter oder Initiator und einem Inhibitor zur Verhinderung einer vorzeitigen Polymerisation. Der Co-Starter kann ggf. auch in der ersten Komponente vorgelegt werden. Da als reaktive organische Flüssigkeit im Sinne der Erfindung jede zur Polymerisation geeignete Flüssigkeit in Frage kommt, sind hier auch Substanzen einbezogen, die bereits jetzt in 2-Pasten-Systemen (z. B. dentale Füllmaterialien) verwendet werden, also meist mehrwertige Makromere wie Bis-GMA und (Di-, Tri-, .., Poly-) Ethylenglycol-Dimethacrylate, mehrarmige PEG-n-(Meth-)Acrylate und analoge Acrylate und Methacrylate und deren Mischungen. Diese Makromere haben bereits eine relativ hohe Viskosität oder können durch gezielte Abmischung der Makromere auf die angestrebte Viskosität eingestellt werden. Weitere Einstellungsmöglichkeiten ergeben sich durch Abmischungen mit anorganischen und organischen Füllstoffen, wie sie aus der Technologie der dentalen Form- und Füllmaterialien bekannt sind. Für die Anwendung als Knochenzemente kommen als Füllstoffe insbesondere solche mit bioaktiven Eigenschaften in Frage, also insbesondere Calcium und/oder Phosphat-haltige Verbindungen.

Angesichts der bevorzugten Verwendung der erfindungsgemäßen Materialien als Knochenzement enthält die zweite Komponente bevorzugt das niedermolekulare Monomer Methylmethacrylat (MMA). Da MMA eine sehr niedrige Viskosität hat, besteht eine bevorzugte Ausführungsform der Erfindung darin, dass die zweite Paste ein in MMA gelöstes Polymer enthält und über die Art und Menge an gelöstem Polymer die Viskosität der zweiten Paste auf die gewünschten Werte eingestellt wird. Obwohl grundsätzlich alle in MMA löslichen Polymere in Betracht kommen, sind solche vom Typ der Polyacrylsäure-Ester, Polymethacrylsäure-Ester, Polystyrol und deren Copolymere bevorzugt. Besonders bevorzugt sind jeweils die Polymere, die in einem 2-Pasten-System der jeweiligen chemischen Zusammensetzung des dispergierten Polymers der ersten Paste weitgehend entsprechen (oder identisch sind) und sich - wenn überhaupt - nur im Copolymeranteil und/oder Molekulargewicht unterscheiden.

Die zweite Komponente des Polymersystems kann ebenfalls als Paste vorliegen, aber auch als Flüssigkeit (Lösung) mit einer Viskosität von vorzugsweise < 200 Pa*s.

Nach einer bevorzugten Ausführungsform der Erfindung liegt der Starter - möglich sind auch Starter und Co-Starter gemeinsam - ebenfalls in einer Form in der Trägerflüssigkeit vor, in der er stabil ist. Typischerweise liegt er als Dispersion in der Trägerflüssigkeit vor oder er ist im dispergierten Polymer enthalten. Der in allen handelsüblichen Knochenzementen enthaltende Radikalstarter BPO wird in diesen beiden Formen eingesetzt und die erfindungsgemäße Technologie ist in beiden Fällen in gleicher Weise umsetzbar. Die Stabilität des Radikalstarters - wie BPO - in wässrigen Dispersionen ist technologisch ein großer Vorteil gegenüber bekannten 2-Pasten-Systemen, die mit gelöstem BPO arbeiten, da BPO ebenso wie andere gebräuchliche Peroxyde in gelöster Form zu spontanem Zerfall neigen und nur eingeschränkt lagerstabil sind. Entsprechende Knochenzemente wie Cortoss® von Orthovita müssen daher gekühlt gelagert werden, um den vorzeitigen Zerfall des BPO zu begrenzen. In der vorliegenden Erfindung wird als Trägerflüssigkeit vorzugsweise eine wässrige Lösung verwendet und darin ist dispergiertes BPO uneingeschränkt lagerstabil. Ähnliches gilt für andere relevante Peroxide.

Nach einer vorteilhaften Ausgestaltung nach Anspruch 5 enthalten die Komponenten des Polymersystems ein Startersystem aus einem 2-Komponenten-Radikalstarter mit einer Starterkomponente und einem Co-Starter, Polymersisationsbeschleuniger oder Initiator als zweite Radikalstarterkomponente. Dabei liegt die Starterkomponente in der ersten Komponente des Polymersystems in ungelöster Form vor und die zweite Radikalstarterkomponente ist in der zweiten Komponente des Polymersystems enthalten.

Vorzugsweise ist die Starterkomponente ein Peroxid und die zweite Radikalstarterkomponente ein tertiäres Amin. Die jeweiligen Komponenten des Stratersystems sind in der jeweiligen Komponente des Polymersystems bei Normalbedingungen bis zur Vermischung der Komponenten im Wesentlichen stabil und lagerfähig.

Sofern das Polymersystem zur Reaktion eines Co-Starters oder Initiators bedarf, liegt dieser vorzugsweise in gelöster Form in der zweiten Komponente vor, kann aber auch suspendiert oder gelöst Bestandteil der ersten Komponente sein, sofern beide Komponenten des Startersystems in der Trägerflüssigkeit der ersten Komponente des Polymersystems nicht miteinander reagieren können, weil z. B eine der beiden oder beide Komponenten des Startersystems in der Trägerflüssigkeit unlöslich sind. Als Co-Starter sind tertiäre Amine bevorzugt, wie sie derzeit in den handelsüblichen Knochenzementen eingesetzt werden. Grundsätzlich sind aber auch hier alle Co-Starter geeignet, die in medizinischen und technischen Polymersystemen praktikabel sind. Die Auswahl der Startersysteme richtet sich dabei nach dem Verwendungszweck des erfindungsgemäßen Polymersystems.

Das erfindungsgemäße Polymersystem enthält den Starter in feinverteilter Form in der Trägerflüssigkeit (wobei er sowohl frei dispergiert vorliegen kann, als auch im Polymer enthalten sein kann, als auch an die Polymeroberfläche adsorbiert sein kann). Zusätzlich kann auch der Co-Starter bereits in der Trägerflüssigkeit enthalten sein, sofern gewährleistet ist, dass beide Substanzen, z. B. wegen mangelnder Löslichkeit in der Trägerflüssigkeit, in dieser Umgebung stabil sind und weder vorzeitig zerfallen noch miteinander reagieren können. Im erfindungsgemäßen Polymersystem sind daher beide Komponenten und die darin enthaltenen Starterkomponenten so lange stabil, bis sie miteinander vermischt werden. Dies gilt zwar auch für konventionelle 2-Pasten-Polymersysteme, allerdings ist dort nur die Reaktionsgeschwindigkeit des Starters abgesenkt, um durch ein genau abgestimmtes Verhältnis von Starter zu Inhibitor (in Verbindung mit Lagerung unter Kühlung) eine für die praktische Anwendung akzeptable Lagerfähigkeit zu erreichen. Ein wesentlicher Aspekt der vorliegenden Erfindung ist daher, dass der Starter in einem Trägermedium (Trägerflüssigkeit der ersten Komponente) dispergiert ist, in dem er ausreichend lagerfähig ist. Bei der Vermischung von erster und zweiter Komponente erfolgt ein Übergang des Starters aus der ersten in die zweite Komponente unter Auflösung der Starterkomponente. Sobald der Starter in der zweiten Komponente des Polymersystems gelöst ist, reagiert er spontan mit dem Co-Starter (sofern erforderlich) und setzt die Polymerisation des Monomers oder Makromers in Gang. Erfindungsgemäß ist der verwendete Starter also in der ersten Komponente unlöslich und in der zweiten Komponente löslich und die Vermischung beider Pasten führt zur Lösung des Starters und Co-Starters in der gleichen Phase, so dass beide Substanzen miteinander reagieren können (falls die Auflösung des Starters in der reaktiven Monomerlösung nicht allein schon zu einer ausreichenden Reaktivität führt).

Das erfindungsgemäße Implantatmaterial aus zwei Komponenten kann so formuliert sein, dass sich die Trägerflüssigkeit der ersten Komponente und die Monomerlösung der zweiten Komponente teilweise oder vollständig ineinander auflösen können, so dass sich zwischen beiden Flüssigkeiten Lösungen oder Mischungen im chemischen Sinne ausbilden. Dies ist beispielsweise der Fall, wenn organische Flüssigkeiten wie Propandiol als Trägerflüssigkeit für die erste Paste verwendet werden und die Monomerlösung der zweiten Paste auf MMA basiert. Vorteile bestehen in dieser Ausführungsform vor allem durch ein anderes mechanisches Verhalten, da sich in diesem Fall eine homogene Matrix ausbildet und kein Porensystem. Ein anderer wichtigerer Aspekt liegt vor, falls das erfindungsgemäße Implantatmaterial als Wirkstoffträger verwendet werden soll. In diesem Fall kann durch eine solch dichte Matrix, die in ihrer Dichte sehr gut eingestellt werden kann, die Wirkstofffreisetzung in weiten Bereichen den Anforderungen angepasst werden

Eine besonders bevorzugte Ausführungsform der Erfindung besteht darin, dass die Trägerflüssigkeit der ersten Komponente und die Monomerlösung der zweiten Komponente im chemischen Sinne nicht miteinander mischbar und/oder nicht oder nur gering ineinander löslich sind und dass daher bei der Vermischung der Komponenten im Wesentlichen eine physikalische Mischung mit der Ausbildung mindestens zweier getrennter Phasen entsteht. Diese Situation ist besonders dann gegeben und besonders bevorzugt, wenn die Trägerflüssigkeit der ersten Komponente eine wässrige Lösung ist und wenn die zweite Komponente auf einer Monomerlösung basiert, wobei die verwendeten Monomere eine geringe Löslichkeit in Wasser aufweisen. Letzteres ist insbesondere gegeben, wenn als Monomer MMA oder andere unpolare Ester der Acrylsäure oder Methacrylsäure (z. B. ButylMethacrylat) oder Styrol oder deren Mischungen zum Einsatz kommen. Dabei wird ausdrücklich hervorgehoben, dass sich die Nicht-Mischbarkeit oder geringe Löslichkeit nur auf die Trägerflüssigkeit und die Monomerflüssigkeiten bezieht und dass sich die in beiden Flüssigkeiten gelösten oder insbesondere suspendierten Substanzen sehr wohl in der jeweils anderen Flüssigkeit lösen können.

Die erfindungsgemäßen Polymersysteme enthalten in der Regel eine oder mehrere Substanzen, die in den jeweiligen Trägerflüssigkeiten suspendiert vorliegen und deren Oberflächeneigenschaften einer stabilen Dispersion entgegenstehen können. Dies gilt beispielsweise für unpolare Polymerpulver, die in einer wässrigen Lösung suspendiert werden oder polare Substanzen, die in der Monomerflüssigkeit suspendiert werden. Es ist daher Teil der Erfindung, dass diese Dispersionen über geeignete oberflächenaktive Substanzen unterstützt und stabilisiert werden. Im Falle der Anwendung als Knochenzement oder ganz allgemein als Implantatmaterial kommen zu diesem Zweck bioverträgliche Tenside zum Einsatz und zwar insbesondere solche, die als pharmazeutische Hilfsstoffe zugelassen und/oder bewährt sind.

Darüber hinausgehend besteht ein bevorzugter erfindungsgemäßer Aspekt der Erfindung im gezielten Zusatz von oberflächenaktiven Substanzen zu einer oder beider (oder aller) Komponenten der erfindungsgemäßen Polymersysteme, die im Falle von Komponenten, deren Flüssigkeiten sich nicht im chemischen Sinne miteinander mischen lassen oder nur eine geringe Löslichkeit ineinander aufweisen, die Ausbildung einer physikalischen Mischung der beiden Pasten unterstützen und diese Mischung zumindest so lange stabilisieren, bis die Polymerisation so weit fortgeschritten ist, dass es nicht mehr zu einer signifikanten oder nachteiligen Entmischung der Komponenten kommen kann. Als oberflächenaktive Substanzen kommen prinzipiell alle für das jeweilige Anwendungsgebiet der erfindungsgemäßen Polymersysteme geeigneten und zugelassenen Stoffe in Betracht, insbesondere - im Falle der Verwendung als Implantatmaterial - alle bioverträglichen Tenside, die auch bisher schon in Pharmazeutika, Kosmetika, Nahrungsmitteln oder Medizinprodukten verwendet werden und/oder für solche Anwendungen zugelassen sind. Für technische Anwendungen gilt Analoges.

Aus der pharmazeutischen Technologie und der Kosmetik ist bekannt, dass in vielen Fällen die Kombination von mehreren Tensiden zur Erzielung der gewünschten Effekte notwendig ist, bzw. dass durch die Kombination verschiedener oberflächenaktiver Substanzen Effekte erreicht werden können, die mit einer einzelnen Substanz nicht realisiert werden können. Es ist daher ein bevorzugter Aspekt der vorliegenden Erfindung, dass mindestens eine oder alle Komponenten des Polymersystems mindestens ein bioverträgliches Tensid enthalten, das die Ausbildung einer physikalischen Mischung der Komponenten unterstützt.

Von der Vielzahl möglicher Tensidzusätze sind vor allem bioverträgliche anionische und nichtionische Tenside bevorzugt. Zum einen gelten diese beiden Gruppen als prinzipiell besser verträglich und zum anderen gibt es unter ihnen nahezu beliebig viele homologe Derivate, mit denen eine gezielte Einstellung von Eigenschaftsspektren möglich ist. Besonders bevorzugt sind im Falle von Implantatmaterialien Formulierungen, in denen zumindest eine Komponente ein bioverträgliches anionisches Tensid enthält, das zumindest eine Carboxyl-, Sulfat- oder Phosphatgruppe enthält, da diese Tenside gleichzeitig als Kristallisationskeime für Knochenmineralien dienen können.

Ebenfalls besonders bevorzugt ist die Verwendung von anionischen Tensiden vom Typ der Seifen, also von Fettsäuren und ihren Alkalisalzen oder Erdalkalisalzen. Unter diesen ist ganz besonders bevorzugt die Verwendung der Ölsäure und deren Natrium-, Kalium-, Ammonium-, Calcium-, Zink- und Magnesiumsalze.

Besonders bevorzugt sind weiterhin Formulierungen, die mehr als ein Tensid enthalten, wobei mindestens eines der Tenside anionisch ist und mindestens ein zweites Tensid nichtionisch ist. Als nichtionische Tenside sollen in diesem Zusammenhang auch solche oberflächenaktive Substanzen angesehen werden, die im technologischen Sprachgebrauch als Co-Tenside bezeichnet werden und beispielsweise aliphatische Alkohole umfassen. Ganz besonders bevorzugt sind solche Tensidkombinationen, mit deren Hilfe die Komponenten der erfindungsgemäßen Polymersysteme beim Vermischen (spontan) Mikroemulsionen ausbilden. Mikroemulsionen haben den großen Vorteil, dass sie sich spontan bilden können, eine reproduzierbare Struktur ausbilden und thermodynamisch stabil sind. Ihre Bildung führt daher zu einer besonders homogenen Matrix der auspolymerisierenden Monomerlösung.

Nach einer bevorzugten Ausführungsform der Erfindung enthält mindestens eine Komponente des Polymersystems mindestens ein bioverträgliches nichtionisches Tensid aus der Gruppe der Polyoxyethylenfettalkoholether (Brij-Typen), Polyoxyethylensorbitan-Fettsäureester (Tween-Typen), Alkylarylpolyetheralkohole (Triton-Typen), Polyoxyethylenpolyoxypropylen-Polymerisate (Random oder Block; Pluronic-Typen). Nach einer weiteren bevorzugten Ausführungsform enthält mindestens eine Komponente des Polymersystems mindestens ein bioverträgliches anionisches Tensid aus der Gruppe der Fettalkoholsulfate, Fettalkoholsulfonate, deren Ethoxylaten, deren jeweiligen Alkalisalzen, der Gruppe der Fettalkoholphosphate (Amphisol-Typen), Fettalkoholphosphonate, deren Ethoxylate und/oder deren Alkalisalze.

Nach Patentanspruch 9 enthält in einer weiteren Ausführungsform der Erfindung die Trägerflüssigkeit oder die Monomerlösung wasserlösliche Monomere oder Makromere, die während oder nach der Polymerisation des Polymersystems ebenfalls zur Polymerisation gebracht werden. Diese wasserlöslichen Monomere oder Makromere polymerisieren vorzugsweise an der Grenzfläche zur Polymermatrix oder direkt in der wässrigen Lösung. Sie erzeugen damit in der wässrigen Phase ein Hydrogel, das entweder der Polymermatrix aufgelagert ist oder aber die wässrige Phase mehr oder weniger homogen ausfüllt. Auf diese Weise können Zusammensetzung, Diffusionsverhalten, Quellungsvermögen, etc. der wässrigen Phase zusätzlich beeinflusst werden. Beispiele für geeignete wasserlösliche Monomere sind Methacrylsäure, HEMA, HPMA, HEMA-Phosphat, Sulfopropyl-Methacrylat, deren Homologe und deren Mischungen. Beispiele für wasserlösliche Makromere sind PEG-mono-Methacrylat, PEG-di-Methacrylat, verzweigte PEG-n-Methacrylate, deren Homologe und deren Mischungen. Diese Aufzählungen haben nur beispielhaften Charakter, umfasst sind alle polymerisierbaren wasserlöslichen Monomere, durch deren Polymerisation Hydrogele entstehen können.

Die Qualität des erfindungsgemäßen Implantatmaterials hängt in den speziellen Anwendungsfällen ganz wesentlich von der Feinabstimmung der beiden Komponenten ab. Die Ausführungsbeispiele zeigen, dass mit relativ einfachen Polymersystemen schon sehr gute Resultate zu erzielen sind. Allerdings können die Anforderungen an das Polymersystem je nach Anwendungsgebiet sehr unterschiedlich sein. Wie bereits weiter oben erwähnt, kann es sinnvoll sein, ein Polymersystem zu schaffen, dass durch eine Phasenseparation oder die Nicht-Mischbarkeit von 2 Phasen ein Porensystem ausbildet. Als Knochenimplantat kann es weiterhin erwünscht sein, dass ein solches Porensystem großenteils oder völlig interkonnektierend ist, damit der umgebende Knochen einwachsen kann. Diese Aufgabe stellt hohe Ansprüche an die Zusammensetzung der beiden Komponenten, so dass über die Viskosität, Strukturviskosität, oberflächenaktive Substanzen, Partikelgrößen der suspendierten Polymere und Hilfsstoffe und Polarität der Flüssigkeiten die Ausbildung des Porensystems gesteuert werden kann. Methoden und Hilfsstoffe zur gezielten Beeinflussung des Verhaltens von Pulver/Flüssigkeits-Mischungen und Emulsionen sind vor allem aus der pharmazeutischen Technologie und der Kosmetik bekannt. In der vorliegenden Erfindung werden diese Technologien erstmals verwendet, um ein neuartiges Implantatmaterial zu realisieren. Besonders beansprucht werden daher Ausführungen des erfindungsgemäßen Polymersystems, in denen die jeweiligen Komponenten durch darin enthaltene gelöste Polymere oder feinteilig suspendierte organische, anorganische oder organomineralische Bestandteile in ihrer Viskosität eingestellt werden und durch die das Mischungs- und Entmischungsverhalten vor und während der Polymerisation beeinflusst werden können. Solche Substanzen sind beispielsweise wasserlösliche Polymere wie lösliche Stärke und Stärkederivate, Cellulosederivate, Kollagen, Gelatine, PEG, PEG-PPG-Copolymere, wasserlösliche modifizierte Polyacrylate/Polymethacrylate, PVP, PVA, etc, die sowohl in der wässrigen Phase gelöst, als auch in der organischen Phase (Monomer) gelöst oder suspendiert vorliegen können. Für die Einstellung der Viskosität der organischen Phase kommen alle Polymere in Betracht, die in der jeweiligen Monomerlösung löslich und mit den Polymerbestandteilen kompatibel sind. Das gilt insbesondere für die jeweils ausgewählten Polymere der ersten Komponente, die sich aber insbesondere im Molekulargewicht von den partikulär eingesetzten Polymeren unterscheiden können. Auch diese Polymere können sowohl direkt als Lösung im Monomer eingesetzt werden oder als besonders fein dispergierte Pulver in der wasserbasierten ersten Komponente, so dass sich diese besonders feinen Partikel im Kontakt mit der Monomerlösung sehr schnell auflösen und einen schnellen Viskositätsanstieg in der Monomerlösung bewirken. Zusätzlich zu den gelösten oder löslichen Substanzen kommen Zusätze in Betracht, die in beiden Komponenten oder Phasen unlöslich sind und die vor allem die Strukturviskosität der Komponenten beeinflussen oder die Phasengrenzen stabilisieren, wie feinstverteilte Silikate und Phosphate, die ggf. zusätzlich modifiziert sein können.

Ein wesentlicher Aspekt der vorliegenden Erfindung ist die gute Mischbarkeit und die minimal invasive Applizierbarkeit der beiden Komponenten des Polymersystems. Versuche haben ergeben, dass sowohl bei manueller Vermischung, besonders aber Vermischung mit 2-Kammersystemen und Statikmischern die Viskosität der beiden Komponenten während der Vermischung nicht zu hoch sein darf und vorteilhafterweise erst nach der Vermischung weiter signifikant ansteigt. Die Viskosität der Komponenten der Polymersystems (vor der Vermischung) liegt daher vorteilhafterweise bei einem Wert von < 200 Pa*s. Bei höheren Viskositäten wird eine gründliche Vermischung der Komponenten erschwert oder eine Ausbringung durch einen Statikmischer beeinträchtigt.

Besonders im Falle der Verwendung der erfindungsgemäßen Polymersysteme als Knochenimplantatmaterialien werden bevorzugt mikrokristalline, nanokristalline oder amorphe Calciumphosphate als mineralische Zuschlagstoffe verwendet. Sie können einerseits die Funktion haben, wie oben genannt, die Viskosität der Pasten zu modifizieren und/oder die Phasengrenzen zwischen der wässrigen Phase und der organischen Phase zu stabilisieren. Darüber hinaus können sie aber auch zusätzlich oder vorwiegend eine biologische Funktion haben, indem sie die Bioaktivität des Implantatmaterials erhöhen und das Einwachsen des Knochens in ein ggf. vorhandenes Porensystem fördern.

Die Bioaktivität des erfindungsgemäßen Polymersystems als Implantatmaterial für Anwendungen im Knochenbereich wird besonders bevorzugt dadurch gefördert, dass zumindest eine der Komponenten Substanzen enthält, die nach Einbringung des Materials an einen Implantationsort die Mineralisation der Oberfläche des Implantatmaterials fördert. Verfahren zur Bioaktivierung von Knochenzementen sind aus DE 10 2005 023 094 A1 bekannt.

Eine bevorzugte Variante besteht darin, dass die in der ersten Komponente des Polymersystems suspendierten Polymere ganz oder teilweise aus Copolymeren bestehen, die anionische Gruppen enthalten oder zu solchen dissoziieren oder hydrolysieren können. Besonders bevorzugt sind solche Copolymere, die Phosphat-, Carboxyl-, Sulfat- oder Silikatgruppen enthalten. Ebenfalls besonders bevorzugt sind Zusammensetzungen, die außer solchen anionischen Copolymeren Calciumsalze und/oder Puffersubstanzen enthalten, die eine hohe Pufferkapazität im alkalischen Bereich haben. Die Calciumsalze und Puffersubstanzen können in jeder der Komponenten enthalten sein. Besonders bevorzugt sind auch Zusammensetzungen, die anionische Monomere in der zweiten Komponente des Polymersystems enthalten und/oder bei denen anionische Copolymere in der zweiten Komponente des Polymersystems gelöst oder suspendiert vorliegen.

Das in DE 10 2005 023 094 A1 beschriebene Verfahren wird im vorliegenden Fall erstmals auf das erfindungsgemäße Implantatmaterial angewendet und hat in diesem Fall den ganz besonderen Effekt, dass der Knochen nicht nur an die Oberfläche des implantierten Knochenzements anwachsen kann, sondern auch in ein sich ausbildendes Porensystem einwachsen kann. Es ist daher besonders bevorzugt, dass die erfindungsgemäßen Polymersysteme - sofern sie als Knochenimplantatmaterialien eingesetzt werden sollen - Substanzen, z. B. Ethylenglycol-Methacrylatphosphat oder Methacrylsäure enthalten, die als Mineralisationskeime für eine Ablagerung von knochenanalogen Mineralien dienen können. Neben den Mineralisationskeimen selbst kann mindestens eine Komponente Zusätze, insbesondere lösliche Calciumsalze und Puffersubstanzen enthalten, die eine Mineralisation fördern können. Die Puffersubstanzen sind in der Lage, den pH-Wert in der direkten Umgebung des Implantatmaterials im angrenzenden wässrigen Medium auf einen neutralen bis basischen Bereich, vorzugsweise pH 7,4 oder darüber, einzustellen oder zu halten.

Vorteilhaft enthält mindestens eine Komponente mindestens eine bioverträgliche Substanz, die unter biologischen Bedingungen und insbesondere unter Bedingungen, wie sie im Knochen herrschen, als Kristallisationskeim für Mineralablagerungen dient und/oder die Ausbildung solcher Mineralablagerungen fördert. Insbesondere polymere Substanzen verfügen dazu mindestens über eine Carboxyl-, Sulfat- und/oder Phosphatgruppe oder eine Siloxangruppe als Substituent.

Das erfindungsgemäße Implantatmaterial und die Verfahren zur Herstellung von daraus bestehenden Knochenimplantatmaterialien erlauben die gezielte Beeinflussung der Bestandteile der sich bildenden Phasen. Insbesondere können im Zuge der Mischung der zwei Komponenten und der anschließenden Polymerisation zwei Phasen gebildet werden, deren Zusammensetzung gezielt gesteuert werden kann. Die wasserbasierte Phase kann als ein Porensystem innerhalb der kontinuierlichen polymerisierten organischen Phase angesehen werden, wobei bei entsprechender Auswahl der Zusammensetzung des Polymersystems und der Verarbeitungsparameter auch die wasserbasierte Phase ein Kontinuum bilden kann und damit ein interkonnektierendes Porensystem bildet. Für das biologische Verhalten ist insbesondere die Zusammensetzung der wasserbasierten Phase von entscheidender Bedeutung. Sie kann neben den essentiellen Komponenten der wässrigen Lösung, die als Trägerflüssigkeit der Polymersuspension der ersten Komponente dient, verschiedene Hilfs-und Wirkstoffe enthalten, die einerseits die Zellaktivität im umgebenden Gewebe beeinflussen und andererseits Wirkstoffe enthalten, die aus diesem Porensystem in die Umgebung des Implantatmaterials freigesetzt werden sollen und deren Freisetzungskinetik über die in der wässrigen Phase enthaltenen Hilfsstoffe effektiv gesteuert werden kann. Wichtigstes Beispiel ist in diesem Zusammenhang die Kombination eines großen Teils der konventionellen Knochenzemente mit Antibiotika, die wie oben erwähnt nur sehr langsam und zu einem sehr geringen Teil aus der Zementmatrix freigesetzt werden. Im Falle der erfindungsgemäßen Polymersysteme können die Antibiotika (und/oder andere geeignete Wirkstoffe) einer der beiden Pasten zugesetzt werden, je nachdem ob eine schnelle oder langsame Freisetzung erwünscht ist (aufgrund der hohen Oberfläche wird allerdings die Freisetzung in jedem Fall sehr viel schneller sein als bei allen konventionellen Knochenzementen). Die vorwiegend relevanten wasserlöslichen Wirkstoffe - wie Antibiotika - werden vorzugsweise der ersten wasserbasierten Paste zugesetzt und befinden sich nach der Polymerisation des Implantatmaterials praktisch ausschließlich in der wässrigen Lösung, die das Porensystem ausfüllt. Ist das Porensystem weitgehend interkonnektierend, können die Wirkstoffe schnell durch Diffusion aus dem Porensystem freigesetzt werden. Auf diese Weise können die Wirkstoffe dem Knochenzement in sehr viel niedrigerer Dosis zugesetzt werden als bisher bei konventionellen Knochenzementen. Zudem entfällt das Risiko der Resistenzentwicklung aufgrund subinhibitorischer Antibiotikakonzentrationen, das für konventionelle antibiotikahaltige Knochenzemente nach wie vor nicht ausgeräumt ist und eine erhebliche Zulassungshürde darstellt.

Die Freisetzung der Wirkstoffe kann darüber hinaus in weiten Bereichen gesteuert werden, indem durch Auswahl geeigneter Salze die Löslichkeit der Wirkstoffe beeinflusst wird, z. B. kann die Löslichkeit der vor allem in Knochenzementen eingesetzten kationischen Antibiotika wie Aminoglycosiden (Gentamicin, Tobramycin) und Glycopeptiden (Vancomycin) durch lipophile und amphiphile Anionen deutlich herabgesetzt werden und so eine verlängerte Freisetzung erreicht werden. Eine weitere sehr effektive Steuerung der Freisetzung erfolgt über die Verwendung von Hilfsstoffen in der wässrigen Phase, die eine freie Diffusion gelöster Wirkstoffe behindern, also praktisch das Porensystem verstopfen. Dies können insbesondere die gleichen Hilfsstoffe sein, die als wasserlösliche Polymere wie lösliche Stärke und Stärkederivate, Cellulosederivate, Kollagen, Gelatine, PEG, PEG-PPG-Copolymere, wasserlösliche modifizierte Polyacrylate/Polymethacrylate, PVP, PVA, etc. die Viskosität der wässrigen Phase beeinflussen, aber auch partikuläre und ggf. quellbare Substanzen, wie Stärkederivate, unlösliches Kollagen, Gelatine (unlöslich) oder mineralische Partikel wie Silikate oder Calciumphosphate, die ggf. zugesetzte Wirkstoffe auch adsorbieren und verzögert wieder abgeben können.

Besonders bevorzugt sind Komponenten, die ein Porensystem ausbilden, das zum überwiegenden Teil oder vollständig interkonnektierend ist. Daraus ergeben sich entscheidende Vorteile für das biologische Verhalten, indem das umgebende Gewebe dadurch die Möglichkeit erhält, tief in das Porensystem einwachsen zu können. Weitere Vorteile ergeben sich für die Wirkstofffreisetzung. In bestimmten Anwendungen und bei bestimmten Formulierungen - die nicht auf die Verwendung als Implantatmaterialien beschränkt sind - ergeben sich auch biomechanische oder technologische Vorteile. Biomechanische Vorteile bestehen insofern, als ein interkonnektierendes Porensystem in einer elastischen Polymermatrix als wirksames hydrodynamisches System fungieren kann, dass auf Belastungen dämpfend reagiert, indem die Flüssigkeit aus dem Porensystem herausgedrückt wird und bei Entlastung wieder zurückströmt. Ein solches Material kann im Knochenbereich vor allem die biomechanische Funktion des spongiösen Knochens der gelenknahen Bereiche und der Wirbelsäule besser nachbilden als bisher verfügbare Materialien.

Die Bildung eines Porensystems im erfindungsgemäßen Implantatmaterial tritt automatisch auf, wenn als Trägerflüssigkeit für die erste Komponente eine Substanz verwendet wird, die nicht mit der Monomerflüssigkeit mischbar ist. In vielen Anwendungen ist die Minimierung der Porosität aus mechanischen Gründen anzustreben, so wie dies auch in konventionellen Knochenzementen versucht wird. Gerade im Einsatzgebiet des osteoporotischen Knochens wird allerdings ebenfalls aus biomechanischen Gründen eine geringere Steifigkeit des Knochenzements angestrebt. Für solche Anwendungen sind Zubereitungen mit einer erhöhten Porosität anzustreben. Untersuchungen haben gezeigt, dass insbesondere bei höheren Porositäten die Druckfestigkeit der Proben zwar deutlich abnimmt, aber immer noch auf einem Niveau liegt, dass gerade für die Auffüllung osteoporotischer Knochen als besonders günstig angesehen wird (siehe: Boger A., Verrier S., Bohner M., Heini P., Schneider E., Injizierbarer poröser Knochenzement für die Vertebroplastik mit physiologisch angepassten mechanischen Eigenschaften, Bern; DGU, 2005). Die Ausführungsbeispiele zeigen auch, dass die in der zitierten Arbeit genannten Ziele mit dem erfindungsgemäßen Imlantatmaterial in besonders guter und zuverlässiger Weise erreicht werden können. Bevorzugt sind daher Zusammensetzungen des erfindungsgemäßen Implantatmaterials, die eine Porosität ergeben (die also nicht mischbare Kombinationen aus Trägerflüssigkeit und Monomerflüssigkeit umfassen). Besonders bevorzugt sind Zusammensetzungen, die eine Porosität von > 10 Volumenprozent im ausgehärteten Implantatmaterial ergeben und ganz besonders bevorzugt sind Zusammensetzungen, die eine Porosität von > 15 Volumenprozent ergeben.

Die Kombination der erfindungsgemäßen Implantatmaterialien mit pharmakologischen Wirkstoffen oder sonstigen Substanzen, die eine Zusatzfunktion ausüben, wurde bereits an verschiedenen Stellen angeführt. An dieser Stelle soll dieser Anspruch wegen seiner Bedeutung für die vorliegende Erfindung zusammengefasst und besonders hervorgehoben werden. Beansprucht werden alle Wirkstoffzusätze, die für die Funktion des erfindungsgemäßen Polymersystems essentiell sind, seine bestimmungsgemäße Anwendung als Medizinprodukt oder technisches Produkt unterstützen können oder seine Verwendung in weitergehende Anwendungsgebiete ausdehnen können. Die bereits vorher genannten und die nachfolgenden Beispiele sollen dabei in keiner Weise als limitierend verstanden werden.

Für die Verwendung der erfindungsgemäßen Implantatmaterialien als Knochenimplantatmaterialien ist die Kombination mit Substanzen von besonderer Bedeutung, die ganz allgemein die bildgebende Diagnostik erleichtern oder erst möglich machen. Dazu zählen die klassischen Röntgenkontrastmittel der Knochenzemente (BaSO₄, ZrO₂) ebenso wie häufig experimentell eingesetzte Metallpulver (Ta, W, Fe, Co oder Legierungen dieser Elemente). Weiterhin sind die Kombination mit nichtionischen Röntgenkontrastmaterialien (meist organische Jod-Verbindungen) oder die Verwendung Jod-haltiger Monomere oder Polymere eingeschlossen, ebenfalls diagnostische Wirkstoffe, die bei anderen bildgebenden Verfahren als Röntgen von Bedeutung sind (Tc, Gd).

Antimikrobielle Wirkstoffe sind als Zusatz zu Knochenzementen seit vielen Jahren etabliert - ihr vorteilhafter Einsatz, bzw. die Vorteile der Erfindung für die Kombination mit Wirkstoffen für die lokale Anwendung geht aus weiter oben angestellten Betrachtungen bereits hervor. Für die erfindungsgemäßen Polymersysteme werden alle Kombinationen mit antimikrobiellen Wirkstoffen beansprucht, insbesondere mit Antibiotika und deren Kombinationen, antiseptischen Substanzen, antimikrobiellen Peptiden und Proteinen, Bakteriophagen, Salzen - insbesondere von Silber oder Wismut, feinstverteiltem metallischen Silber, und antimikrobiell wirkenden Kombinationen dieser Wirkstoffe untereinander und mit anderen Wirkstoffen. Beansprucht wird ebenfalls die Kombination mit antiproliferativ, cytostatisch, immunsuppressiv oder antünflammatorisch wirkenden Substanzen.

Besonders bevorzugt ist weiterhin die Kombination mit Wirkstoffen, die spezifisch oder unspezifisch den Knochenstoffwechsel beeinflussen können. Dazu zählen viele Vitamine, insbesondere Vitamin D, weiterhin Substanzen, die eine inhibierende Wirkung auf Osteoklasten und ganz allgemein auf Entzündungszellen haben, oder spezifische Stoffwechselreaktionen der Osteoklasten und Entzündungszellen hemmen, wie insbesondere deren Produktion und Sekretion von Säure (z. B. Protonenpumpen-Inhibitoren, Bisphosphonate). Insbesondere zählen dazu auch Wirkstoffe, die eine Differenzierung von Osteoblasten aus Vorläuferzellen stimulieren wie die Wachstumsfaktoren aus der TGFβ-Familie, besonders BMP 2 und BMP 7, sonstige Wachstums- uns Differenzierungsfaktoren, sowie Wirkstoffe, die ganz allgemein lokal die Stoffwechselleistung erhöhen können (wie PTH, PTH-Fragmente, IGF, und andere anabole Hormone) und solche, die die Bildung von Blutgefäßen in der Implantatumgebung fördern (wie FGF oder VEGF).

Viele der genannten - und weiterer für den Knochen relevanter - Wirkstoffe sind mit den bisherigen Knochenzementen nicht in sinnvoller Weise kombinierbar, weil sie die Polymerisationsbedingungen nicht aushalten, nicht oder nicht in ausreichender Menge freigesetzt werden können, bedenkliche Langzeitfolgen haben könnten oder aus wirtschaftlichen Gründen wegen des überwiegenden Teils nicht freisetzbaren Wirkstoffs für eine Kombination mit konventionellem Knochenzement ungeeignet sind. Viele dieser Wirkstoffe werden in Kombination mit dem erfindungsgemäßen Polymersystem überhaupt erstmals in pharmakologisch sinnvoller Weise mit einem Implantatmaterial und/oder einem Knochenzement kombinierbar.

Die erfindungsgemäßen Implantatmaterialien sind in einfacher Weise herstellbar. Die Anmischung ist bereits in einfachen Mischbechern mit gutem Ergebnis möglich. Im Sinne einer einfacheren Anmischung und reproduzierbarere Qualität des Mischergebnisses ist die Verarbeitung des Polymersystems in einem vorgefüllten und fertig konfektionierten Behältnis und Mischsystem eine besonders bevorzugte Darreichungsform. Auch hier besteht ein großer Vorteil gegenüber konventionellen Knochenzementen, die in solchen einfachen, üblicherweise als Doppelkammerspritzen ausgeführten Mischsystemen nicht verarbeitet werden können. Geeignete Doppelkammerspritzen für die erfindungsgemäßen Polymersysteme sind z. B. von der Firma Mixpac® erhältlich. Der Anspruch ist aber nicht auf diese Fabrikate beschränkt, sondern umfasst alle Systeme, inkl. solcher, die mechanisch angetrieben werden und z. B. bei Zahnärzten oder Zahntechnikern gebräuchlich sind und dort für die Mischung hochviskoser Pasten (z. B. für Abdruckmassen) verwendet werden. Die gebräuchlichen Mischungsverhältnisse reichen von 1:1 über 1:2, 1:4 bis 1:10, können aber auch auf andere Relationen eingestellt werden. Für die erfindungsgemäßen Polymersysteme sind besonders die Mischungsverhältnisse 1:1, 1:2 und 1:4 geeignet und bevorzugt.

Der Bereich der Knochenimplantatmaterialien stellt ein bevorzugtes Anwendungsgebiet der erfindungsgemäßen Implantatmaterialien dar, in dem die prinzipiellen Vorteile des erfindungsgemäßen Implantatmaterials besonders offenkundig sind und besonders ausgeprägt zum Tragen kommen. Wie eingangs dargestellt haben die konventionellen Knochenzemente trotz ihrer sehr weiten Verbreitung in einigen Aspekten gravierende Schwächen, die mit den erfindungsgemäßen Polymersystemen zumindest teilweise überwunden werden können. Sie seien nachfolgend kurz zusammenfassend dargestellt:
■ Im Gegensatz zur aufwendigen Mischung der Pulver in konventionellen Knochenzementen lassen sich die Pasten in einfacher Weise und auf kostengünstigen Maschinen (z. B. Planetenrührwerk) zubereiten. Eine Gefahr der Entmischung von Pulvern unterschiedlicher Dichte und Körnung besteht nicht. Die Pasten lassen sich ohne Gefahr einer Einmischung von Luftblasen problemlos miteinander vermischen. Es entstehen keine Makrodefekte, die bei konventionellen Knochenzementen ein erhebliches Problem für die mechanische Leistungsfähigkeit darstellen.
■ Die aus konventionellen Knochenzementen bekannte und besonders nachteilige Schwindung tritt bei den erfindungsgemäßen Knochenzementen in deutlich geringerem Umfang auf und kann je nach Rezeptur fast völlig unterdrückt werden. Da bei konventionellen Knochenzementen die Schwindung eine Funktion des Monomergehalts ist und die erfindungsgemäßen Polymersysteme mit sehr viel weniger Monomer auskommen können, liegt hier auch die Schwindung deutlich niedriger.

■ Ebenso wie die Schwindung ist auch die Exothermie eine Funktion des Monomergehalts und entsprechend ist auch sie in gleicher Weise reduziert. Zudem unterstützt in wasserbasierten Pasten das eingebrachte Wasser mit seiner hohen Wärmekapazität als wirksames Mittel, den Temperaturanstieg während der Polymerisation auf ein verträgliches Maß zu reduzieren.
■ Die erfindungsgemäßen Polymersysteme erreichen vielfach nicht die absoluten mechanischen Werte der konventionellen Knochenzemente für Druck- und Biegefestigkeit, jedoch liegen sie vielfach (je nach Formulierung) oberhalb der Normwerte. Andererseits zeigen die erfindungsgemäßen Polymersysteme ein mechanisches Verhalten - und aus diesem Grund werden sie primär hier vorgeschlagen - dass sie besonders für die Auffüllung spongiöser Knochendefekte prädestiniert. Insbesondere ist ihre Steifigkeit deutlich reduziert, so dass sie als Implantat den angrenzenden Knochen weniger schädigen. Zudem zeigen spezielle Formulierungen ausgeprägte Dämpfungseigenschaften. Überraschenderweise zeigen allerdings einige der getesteten Formulierungen auch statische Festigkeitswerte, die denen der besten konventionellen Knochenzemente nicht nachstehen. Eine Eignung zur Fixierung von Gelenkimplantaten ist damit ebenfalls gegeben.
■ Die überlegene Freisetzung von Wirkstoffen wurde bereits ausführlich erwähnt.
■ Die Bioaktivität ist bei den erfindungsgemäßen Polymersystemen gleich in mehrfacher Hinsicht überlegen. Einmal durch die vielfältige Kombinationsmöglichkeit mit bioaktiven Wirkstoffe, zweitens durch die geringere Härte, die eine biomechanisch Induktion der Knochenheilung fördert, drittens durch die mögliche Einbringung von bioaktiven Mineralien und Kombination mit Mineralisationskeimen und viertens durch die mögliche Realisierung eines interkonnektierenden Porensystems, das ein Einwachsen von Knochen und Blutgefäßen bis tief in die Zementmatrix erlaubt.

Auf Grund dieser Vorteile ist die Verwendung der erfindungsgemäßen Polymersysteme zur Herstellung von Knochenersatzmaterial, Knochenzementen, Knochenklebern und implantierbaren Wirkstoffträgern besonders bevorzugt. Knochenzemente sind entsprechend des erfindungsgemäßen Implantatmaterials aufgebaut und an die spezifischen Anwendungen angepasst. Knochenersatzmaterialien sind Zubereitungen aus dem erfindungsgemäßen Implantatmaterial, z. Bsp. zur Auffüllung von Knochendefekten. In diesem Fall kann es vorteilhaft sein, das Knochenersatzmaterial aus dem erfindungsgemäßen Implantatmaterial vor der Implantation in den Körper in Form eines ausgehärteten soliden Werkstücks zu formen und anschließend in den Knochendefekt einzubringen/zu implantieren.

Knochenkleber sind Zubereitungen aus dem erfindungsgemäßen Implantatmaterial, z. B. zur Verbindung und Befestigung von Knochenfragmenten nach Knochenbrüchen oder zur Befestigung von z. B. metallischen oder keramischen oder anderen polymeren Implantatmaterialien am oder im Knochen. In dieser Funktion enthalten die Knochenkleber neben den erfindungsgemäßen Implantatmaterialien adhäsionsförderde Substanzen, die aus dem Dentalbereich als Adhäsionspromotoren bekannt sind und/oder Substanzen wie Polyacrylate, anionische Monomere, und daraus hergestellte Polymere und/oder Copolymere. Diese Substanzen werden im Dentalbereich u. a. zur Verbesserung der Haftung von Füllungsmaterialien an der Zahnsubstanz verwendet (die mit der Knochensubstanz große Ähnlichkeit hat). Mit den erfindungsgemäßen Implantatmaterialien sind sie besonders vorteilhaft und vielseitig kombinierbar, da sie sowohl mit der ersten Komponente des Polymersystems, als auch mit der zweiten Komponente des Polymersystems in weiten Konzentrationsbereichen kombiniert werden können. Dies unterscheidet die erfindungsgemäßen Implantatmaterialien von konventionellen Zubereitungen, weil insbesondere die in dieser Hinsicht besonders wirksamen, geladenen oder stark polaren Adhäsionspromotoren keine oder nur eine geringe Löslichkeit in konventionellen Knochenzementen (bzw. deren Monomerlösungen) haben. Besonders bevorzugt ist die Kombination mit Polyacrylsäure, Copolymeren der Acrylsäure und/oder Methacrylsäure, insbesondere Copolymeren mit Maleinsäure, und deren Salzen. Ebenfalls besonders bevorzugt sind in dieser Hinsicht Kombinationen mit Phosphatgruppen-haltigen (ethylenisch ungesättigten) Monomeren und/oder daraus hergestellten Polymeren oder Copolymeren. Ganz besonders bevorzugt sind Zumischungen der vorgenannten Adhäsionspromotoren in einer Konzentration von 0,1 bis 50% bezogen auf die Gesamtmasse des Implantatmaterials. Die Zugabe der Adhäsionspromotoren bewirkt eine stärkere Anhaftung des Implantatmaterials am Knochengewebe.

Wirkstoffträger sind Zubereitungen aus erfindungsgemäßen Implantatmaterialien, die pharmakologische Wirkstoffe in Konzentrationen enthalten, die eine therapeutische Funktion im menschlichen oder tierischen Organismus bewirken können. Diese Wirkstoffe werden nach der Implantation aus dem Implantatmaterial freigesetzt. Bevorzugt ist daher die Kombination mit Wirkstoffen, die eine direkte lokale Wirkung entfalten, die also direkt auf das Gewebe in der Umgebung des Implantatmaterials wirken. Bevorzugt ist die Kombination mit Wirkstoffen, die den Knochenstoffwechsel stimulieren und lokalen Entzündungen entgegenwirken. Besonders bevorzugt sind Wirkstoffträger auf der Basis der erfindungsgemäßen Implantatmaterialien, die antimikrobielle Wirkstoffe wie Antibiotika enthalten.

Eine besonders bevorzugte Anwendung der erfindungsgemäßen Polymersysteme besteht in der Auffüllung von Knochendefekten und in der Augmentation von osteoporotischem Knochen. In den allermeisten Fällen erfolgt diese Applikation in minimal invasiver Operationstechnik.

Ein typisches Beispiel dafür sind die verschiedenen Verfahren der Vertebroplastie, die in den letzten Jahren immer populärer geworden sind und deren klinische Wirksamkeit immer besser dokumentiert wird. Es ist daher davon auszugehen, dass diese Behandlungstechniken weiter ausgearbeitet werden und die behandelten Fälle stark zunehmen werden. Die Weiterentwicklung der Methoden der Vertebroplastie (inkl. der sogenannten Kyphoplastie) sind ganz wesentlich von der Verfügbarkeit verbesserter Augmentationsmaterialien abhängig. Die bisherigen Knochenzemente sind nur bedingt geeignet, auch wenn einige Produkte speziell für die Vertebroplastie angeboten werden, sind sie doch nach wie vor nur konventionelle Knochenzemente mit geringfügig modifizierter Viskosität, Aushärtekinetik und erhöhtem Gehalt an Röntgenkontrastmaterial. Die erfindungsgemäßen Polymersysteme sind prinzipiell in diesen Anwendungen überlegen, weil sie in ihrer Handhabung deutlich einfacher sind, geringere Schwindung, geringere Wärmeentwicklung, höhere Bioaktivität und bessere biomechanische Kompatibilität mit dem Knochen aufweisen.

Für die klinisch erfolgreiche Anwendung müssen sie mit geeigneten Applikationssystemen zur Einbringung des Polymersystems in den Knochen kombiniert werden. Dazu können im einfachsten Fall einfache Spritzensysteme verwendet werden, wie sie auch heute noch in der Vertebroplastie eingesetzt werden. Zweckmäßiger ist allerdings die Mischung des Polymersystems in der Doppelkammerspritze und einem Statikmischer und die Kombination mit einer Injektionskanüle bzw. einem geeigneten Rohr, das bis an die Implantationsstelle reicht. Vorteilhaft ist hier insbesondere, dass das Polymersystem erst bei der Extrusion gemischt wird, also in einfacher Weise mit einer Zementkartusche mehrere Injektionen gemacht werden können und es keiner zeitlich abgestimmten Vorarbeit bedarf, weil das Polymersystem gleich anwendungsbereit aus der Spritze kommt.

Eine besonders bevorzugte Anwendung ist das als Kyphoplastie bekannte Verfahren, bei dem zunächst der osteoporotische Knochen des Wirbelkörpers mit einem Ballon aufgedehnt und in der Umgebung komprimiert wird und anschließend in den erzeugten Hohlraum ein Knochenzement eingebracht wird. Gerade bei dieser Anwendung ist die Kombination des Applikationsinstrumentariums mit dem erfindungsgemäßen Polymersystem besonders vorteilhaft, weil in diesem Fall ein vorteilhaftes Applikationssystem mit einem vorteilhaften Füllmaterial kombiniert werden kann. Es werden daher alle Kombinationen des erfindungsgemäßen Polymersystems mit Applikationssystemen beansprucht, die geeignet sind, das erfindungsgemäße Polymersystem in Knochendefekte, Frakturspalten, osteoporotischen Knochen, Knochentumore oder auf andere Art rarifizierte Knochenstrukturen in minimal invasiver Art und Weise einzubringen. Ebenfalls werden alle Zusätze beansprucht, die an ein Mischsystem vom Typ der genannten Doppelkammerspritzen angebaut werden können oder mit einem solchen System verbunden werden zu dem Zweck, um mit deren Hilfe die erfindungsgemäßen Polymersysteme an den Zielort zu applizieren.

Die erfindungsgemäßen Implantatmaterialien lassen sich in besonders vorteilhafter Weise intraoperativ handhaben, also für die Einbringung in den Körper präparieren. Das unterscheidet sie grundsätzlich von konventionellen Knochenzementen, die aus Pulver und Flüssigkeit angemischt werden. Gleichzeitig erlauben die Materialeigenschaften die einfache und zuverlässige minimal invasive Applikation über eine Kanüle. Die freie Kombinierbarkeit der ersten und der zweiten Komponente des Polymersystems in einem sehr breiten Mischungsverhältnis (z. B. 1:1, 1:2, 1:4) ermöglicht auch die Verwendung der erfindungsgemäßen Zusammensetzungen für die Herstellung von Knochenklebern, Knochenersatzmaterialien und implantierbaren Wirkstoffträgern neben der Anwendung von Knochenzementen.

Anhand nachfolgender Ausführungsbeispiele wird die Erfindung näher erläutert:

### Ausführungsbeispiel 1

Beispiel für den prinzipiellen Aufbau des erfindungsgemäßen Polymersystem:
a) Komponente 1: 30 g Polymerpulver Degacryl® 6658 F (Co-Polymer aus PMMA und PMA (94:6) enthaltend 1,5% BPO, Partikelgröße ca. 45 µm); 15 ml Wasser (demin.); 0,45 g Tensid (Tween 80); 0,3 g Carboxymethylstärke (Typ PO) .
b) Komponente 2: 10 ml Methylmethacrylat (MMA) enthaltend 0,5% DMPT;

Ausführungsbeispiel 1 zeigt in einem einfach aufgebauten Versuch das Funktionsprinzip des erfindungsgemäßen Polymersystems. Als erste Komponente dient ein handelsübliches aus der Dentaltechnik bekanntes Polymer der Firma Degussa (Degacryl® 6658 F). Dieses Polymer wird mit der wässrigen Lösung eines Tensids - zur besseren Mischbarkeit des Polymerpulvers mit der wässrigen Lösung und anschließenden Mischung der Paste mit der Monomerflüssigkeit - und eines Polymers (Carboxymethylstärke) - zur Erhöhung der Viskosität - zu einer makroskopisch homogenen Paste vermischt. Die so erhaltene Paste zeigt gute Lagereigenschaften, indem sie nach Aufbewahrung in einem verschlossenen Glasgefäß unter Normalbedingungen innerhalb von 2 Monaten keine erkennbaren Veränderungen zeigt. Die zweite Komponente besteht in diesem Beispiel aus konventionellem Knochenzement-Monomer. Das Startersystem besteht aus BPO/DMPT und ist wie bei konventionellem Knochenzement auf die beiden Komponenten verteilt.
Die Mischung der Komponenten 1 und 2 erfolgt im Mischbecher mit einem Spatel. Nach einer Mischdauer von einer Minute wird eine homogene Mischung erhalten, die in ca. 5 Minuten unter Erwärmung aushärtet. Zylindrische Formkörper aus dieser Mischung mit der Abmessung 10 mm Durchmesser und 20 mm Höhe erreichen nach einer Inkubation über Nacht in simulierter Körperflüssigkeit bei 37°C eine Druckfestigkeit von 35 - 50 MPa.

Die Ergebnisse zeigen, dass bei einer Vermischung der wasserbasierten Paste aus Polymerpulver mit einer Monomerflüssigkeit, die jeweils aus der Zusammensetzung konventioneller Knochenzemente abgeleitet sind, eine Polymerisationsreaktion ausgelöst wird, die darauf schließen lässt, dass sich das in Wasser suspendierte Polymerpulver mit der Monomerlösung verbindet, dass sich das Polymerpulver teilweise auf- oder anlöst, dass sich das im Polymerpulver enthaltende BPO dabei aus diesem herauslöst und im Monomer in Lösung geht, dass in der Monomerlösung durch das Zusammentreffen der beiden Starterkomponenten eine Polymerisationsreaktion ausgelöst wird und dass durch die Polymerisation eine innige Verbindung zwischen Polymerpulver und auspolymerisierendem Monomer gebildet wird. Der erhaltene Feststoff erreicht eine hohe Druckfestigkeit, die zwar geringer liegt, als die konventioneller Knochenzemente, allerdings ist hierbei zu berücksichtigen, dass der erhaltene Feststoff eine Porosität von > 30% aufweist (siehe auch Beispiel 3).

Überraschenderweise härtet die beschriebene Rezeptur deutlich schneller aus, als eine vergleichbare Rezeptur aus pulverförmigem Degacryl® 6658 F, das mit dem gleichen Monomer angemischt wird und ca. 12 Minuten bis zur Aushärtung benötigt.

### Ausführungsbeispiel 2

Beispiel zur Herstellung eines erfindungsgemäßen Knochenzements auf der Basis von Palacos R (Heraeus-Kulzer)

Komponente 1: 40 g Pulver des Knochenzements Palacos® R der Firma Heraeus-Kulzer enthaltend eine Mischung aus PMMA/PMA Co-Polymeren, Röntgenkontrastmittel (Zirkondioxid) und Benzoylperoxid (BPO) werden mit 15 ml einer wässrigen Lösung enthaltend 2,5% Tensid (Tween® 80) und 2% Carboxymethylstärke (Typ PO) zu einer Paste vermischt. Die Paste ist makroskopisch homogen und entmischt sich nicht unter Normalbedingungen.

Komponente 2: 10 ml Methylmethacrylat (MMA) enthaltend 0,5% DMPT

Mischung der Komponenten 1 und 2 im Mischbecher. Nach kurzer Mischdauer mit dem Spatel wird eine homogene Mischung erhalten, die in ca. 5 Minuten unter Erwärmung aushärtet. Zylindrische Formkörper aus dieser Mischung mit der Abmessung 10 mm Durchmesser und 20 mm Höhe erreichen nach einer Inkubation über Nacht in simulierter Körperflüssigkeit bei 37°C eine Druckfestigkeit von > 50 MPa.

Beispiel 2 zeigt, dass die Ergebnisse aus Beispiel 1 auch auf die Rezeptur eines handelsüblichen konventionellen Knochenzements übertragen werden können. Überraschenderweise härtet auch diese Rezeptur deutlich schneller aus, als eine vergleichbare Rezeptur aus dem pulverförmigen Knochenzement Palacos® R, der mit dem gleichen Monomer angemischt wird und ca. 13 Minuten bis zur Aushärtung benötigt.

### Ausführungsbeispiel 3

Implantatmaterial, das nach der Aushärtung einen Feststoff mit interkonnektierendem Porensystem ergibt:
a) Komponente 1: 30 g Polymerpulver Degacryl® 6658 F (Co-Polymer aus PMMA und PMA (94:6) enthaltend 1,5% BPO, Partikelgröße ca. 45 µm); 15 ml Wasser (demin.); 0,45 g Tensid (Tween 80); 0,3 g Carboxymethylstärke (Typ PO) .
b) Komponente 2: 7 ml Methylmethacrylat (MMA) enthaltend 0,5% DMPT;

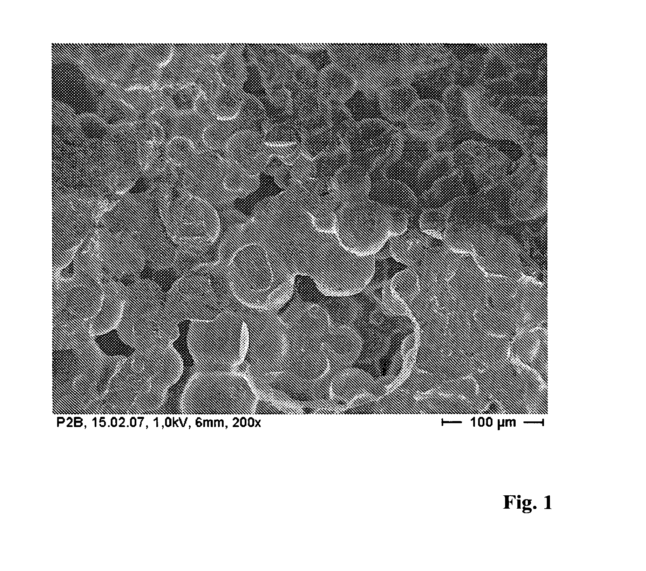

Die Mischung der Komponenten 1 und 2 erfolgt im Mischbecher mit einem Spatel. Nach einer Mischdauer von einer Minute wird eine homogene Mischung erhalten, die in ca. 5 Minuten unter Erwärmung aushärtet. Zylindrische Formkörper aus dieser Mischung mit der Abmessung 10 mm Durchmesser und 20 mm Höhe erreichen nach einer Inkubation über Nacht in simulierter Körperflüssigkeit bei 37°C eine Druckfestigkeit von 30-40 MPa. Kugelförmige Formkörper aus dieser Mischung von 20 mm Durchmesser werden nach kompletter Aushärtung über Nacht in simulierter Körperflüssigkeit im Trockenschrank bei 37°C getrocknet und zeigen dabei einen Gewichtsverlust von ca. 35%, was der rechnerischen Porosität entspricht. Nach anschließender erneuter Inkubation in simulierter Körperflüssigkeit für 24 Stunden nehmen die Formkörper wieder die gleiche Flüssigkeitsmenge auf. Diese Ergebnisse belegen eine interkonnektierende Porosität. Fig. 1 zeigt dazu eine Aufnahme im Raster-Elektronenmikroskop.

### Ausführungsbeispiel 4

### Applikationsset

Das Applikationsset besteht aus einer Doppelkammerspritze der Firma Mixpac® und 2 Komponenten gemäß Ausführungsbeispiel 1.

20 ml der Komponente 1 nach Beispiel 1 wird in die größere Kammer einer Doppelkammerkartusche (4:1) der Firma Mixpac abgefüllt und frei von Luftblasen mit einem Kolben verschlossen. Anschließend wird die kleinere Kammer mittels einer Spritze mit Monomer, in dem zuvor 5% eines PMMA-Polymers (MW 230.000) homogen gelöst wurde, komplett gefällt, nachdem der Kolben auf die gleiche Position wie in der größeren Kammer gebracht wurde. Die Doppelkammerkartusche wird danach mit einem Verschlussstopfen verschlossen. Zum Zwecke der Mischung und Austragung wird die Kartusche in ein Austraggerät eingelegt und der Verschlussstopfen entfernt und durch einen Statikmischer ausgetauscht. Anschließend werden die Kolben der Kammern gleichmäßig durch das Austraggerät nach von gedrückt und sowohl die Paste, als auch die Monomerlösung durch den Statikmischer gedrückt, wobei eine innige Vermischung erfolgt. Der erste Milliliter Extrudat wird als unzureichend homogen gemischt verworfen, das weitere Extrudat ist homogen gemischt und härtet wie das manuell gemischte Material in ca. 5 min aus. Mittels der Doppelkammerspritze lässt sich das Material gut applizieren.

### Ausführungsbeispiel 5

Implantatmaterial, das nach der Aushärtung einen Feststoff mit interkonnektierendem Porensystem ergibt in Abhängigkeit vom gewählten Mischungsverhältnis der ersten und zweiten Komponente des Polymersystems und Präparation im 2-Kammer-Mischsystem:
c) Komponente 1: 20g Polymerpulver Degacryl® 6658 F (Co-Polymer aus PMMA und PMA (94:6) enthaltend 1,5% BPO, Partikelgröße ca. 45 µm); 5g Röntgenkontrastmittel (Zirkondioxid); 11ml Wasser (demin.) enthaltend 2,5% Tensid (0,275g Tween 80); 0,4g Polyethylenoxid (Polyox 574); 0,4g Natrium-Oleat, 0,5g Calciumchlorid.
d) Komponente 2: Methylmethacrylat (MMA) enthaltend 0,5% DMPT mit 20% Degacryl M546 und 2% Emulgator (Ölsäure)

Die Mischung der Komponenten 1 und 2 erfolgt im 2-Kammer-Mischsystem, wobei die Angabe des Mischungsverhältnisses das Verhältnis von Komponente 1 zu Komponente 2 beschreibt. Die Mischung und Austragung der Komponenten im 2-Kammermischsystem erfolgt wahlweise mit Hilfe eines Dispensers oder mit Hilfe eines Stössels von Hand. Durch Ausspritzen des Pastenmaterials durch einen Statikmischer wird eine homogene Mischung erhalten, die in ca. 5 Minuten unter Erwärmung aushärtet. Zylindrische Formkörper aus dieser Mischung mit der Abmessung 6 mm Durchmesser und 12 mm Höhe erreichen nach einer Inkubation über Nacht in simulierter Körperflüssigkeit bei 37°C eine Druckfestigkeit von ca. 30-50 MPa, abhängig vom jeweiligen Mischverhältnis.

| **Mischsystem 4:1, Dispenser** | | **Mischsystem 2:1, Dispenser** | | **Mischsystem 1:1, Handstössel** | |
|---|---|---|---|---|---|
| **10ml-Doppelkammer-Kartusche, Mischaufsatz ∅ 3,2 mm x L16 mm** | | **25ml-Doppelkammer-Kartusche, Mischaufsatz ∅ 4,2mm x L 12mm** | | **5ml-Doppelkammerspritze, Mischaufsatz ∅ 2,5 mm x L16mm** | |
| Druckfestigkeit: 28,92 +/- ,24 MPa | | Druckfestigkeit: 38,83 +/- ,19 MPa | | Druckfestigkeit: 48,47 +/- 1,89 MPa | |
| Porosität: ca. 23% | | Porosität: ca. 19% | | Porosität: ca. 16% | |
| ***Verarbeitung:*** | | ***Verarbeitung:*** | | ***Verarbeitung:*** | |
| • | gut spritzbar | • | gut spritzbar | • | gut spritzbar |
| • | komplette Kartuschen-Entleerung möglich | • | komplette Kartuschen-Entleerung möglich | • | komplette Kartuschen-Entleerung möglich |
| • | homogen gemischte Zementpaste | | | • | homogen gemischte Zementpaste |
| | | • | homogen gemischte Zementpaste | | |
| • | Pastenviskosität unmittelbar nach Mischung: ● | | | • | Pastenviskosität unmittelbar nach Mischung: ○ |
| | | • | Pastenviskosität unmittelbar nach Mischung: ● | | |

Legende zur Beschreibung der Pastenviskosität:
- Pastenviskosität ●: hohe Viskosität der Zementmischung, Formstabilität der gespritzten Zementmischung - Strang der Zementmischung behält Form nach Spritzen
- Pastenviskosität ●: mittlere Viskosität der Zementmischung, Strang der Zementmischung zerläuft leicht, Strangform noch erkennbar
- Pastenviskosität ○: geringe Viskosität der Zementmischung, Strang der Zementmischung verläuft, Einzelstränge verbinden sich

Mechanische Kennwerte nach Probenpräparation im Mischbecher:

| **Mischverhältnis 4:1** | **Mischverhältnis 2:1** | **Mischverhältnis 1:1** |
|---|---|---|
| **Druckfestigkeit: 34,17 +/- 1,55 MPa** | **Druckfestigkeit: 47,17 +/- 4,84 MPa** | **Druckfestigkeit: 53,78 +/- 3,26 MPa** |

### Abkürzungen

- Bis-GMA: Bisphenol A diglycidylether methacrylate (Bis-GMA).
- BMP: Bone Morphogenetic Protein
- BPO: di-Benzoylperoxid
- DMPT: Dimethyl-p-Toluidin
- DMSO: Dimethylsulfoxid
- FGF: Fibroblast Growth Factor
- HEMA: Hydroxyethyl-Methacrylat
- HPMA: Hydroxypropyl-Methacrylat
- IGF: Insulin-like Growth Factor
- MMA: Methyl-Methacrylat
- PEG: Polyethylenglycol
- PEO: Polyethylenoxid
- PPG: Polypropylenglycol
- PVA: Polyvinylalkohol
- PVP: Polyvinylpyrrolidon
- PMMA: Polymethylmethacrylat
- PTH: Parathormon
- TGF: Transforming Growth Factor
- VEGF: Vascular Endothelial Growth Factor

## Patentansprüche

1. Implantatmaterial auf Basis eines Polymersystems aus mindestens 2 Komponenten, die bei Vermischung miteinander reagieren und einen polymerbasierten Feststoff bilden, wobei die erste Komponente des Polymersystems mindestens ein bioverträgliches Polymerpulver und eine Starterkomponente zur Auslösung einer Polymerisationsreaktion bei Vermischung enthält und wobei die zweite Komponente des Polymersystems mindestens eine reaktive organische Flüssigkeit oder eine Lösung oder eine Suspension einer reaktiven organischen Flüssigkeit und eines bioverträglichen Polymeren enthält, **dadurch gekennzeichnet, dass** die erste Komponente des Polymersystems eine lagerstabile Paste ist, die das Polymerpulver und die Starterkomponente in Kombination mit einer Trägerflüssigkeit enthält, wobei sich unter Normalbedingungen in der Trägerflüssigkeit das Polymerpulver und die Starterkomponente nicht auflösen, sondern suspendiert vorliegen und die Starterkomponente bis zur Vermischung mit der zweiten Komponente des Polymersystems stabil bleibt.

2. Implantatmaterial nach Anspruch 1, **dadurch** charakterisiert, dass die bioverträgliche Polymerkomponente aus Homo- oder Co-Polymerisaten von Acryl-, Methacrylsäureestern, Styrol- , Vinyl-Derivaten oder deren Mischungen ausgewählt ist und/oder dass die Trägerflüssigkeit ausgewählt ist aus Wasser, wässrigen Lösungen, Glycerin, Propandiol, niedermolekularem PEG, PEG-PPG-Copolymeren, DMSO, Methyl-Pyrrolidon, bioverträgliche Ölen oder deren Mischungen untereinander oder deren Mischungen untereinander mit anderen Substanzen.

3. Implantatmaterial nach einem der Ansprüche 1 oder 2, **dadurch gekennzeichnet, dass** die zweite Komponente als reaktive organische Flüssigkeit Methylmethacrylat oder homologe Ester der Methacrylsäure oder deren Mischungen enthält.

4. Implantatmaterial nach einem der Ansprüche 1 bis 3, **dadurch gekennzeichnet, dass** das Polymer der zweiten Komponente ein Polyacrylsäure-Ester oder ein Polymethacrylsäure-Ester oder ein Polystyrol oder deren Copolymer ist.

5. Implantatmaterial nach einem der Ansprüche 1 bis 4, **dadurch** gekenntzeichnet, dass das Startersystem ein 2-Komponenten- Radikalstarter ist, wobei eine Komponente des Startersystems in ungelöster Form in der Trägerflüssigkeit in der ersten Komponente des Polymersystems und die zweite Komponente des Startersystems als Co-Starter, Polymerisationsbeschleuniger oder Initiator in der zweiten Komponente des Polymersystems enthalten ist.

6. Implantatmaterial nach einem der Ansprüche 1 bis 5, **dadurch gekennzeichnet, dass** die erste oder die zweite oder beide Komponenten des Polymersystems wasserlösliche Polymere und/oder Antibiotika enthalten.

7. Implantatmaterial nach einem der Ansprüche 1 bis 6, **dadurch gekennzeichnet, dass** die Trägerflüssigkeit der ersten Komponente des Polymersystems und die reaktive organische Flüssigkeit der zweiten Komponente des Polymersystems im chemischen Sinne nicht miteinander mischbar bzw. nicht oder nur gering ineinander löslich sind und dass bei der Vermischung dieser Komponenten eine physikalische Mischung entsteht.

8. Implantatmaterial nach Anspruch 7, **dadurch gekennzeichnet, dass** mindestens eine Komponente des Polymersystems mindestens ein anionisches und/ oder nichtionisches bioverträgliches Tensid enthält, das die Ausbildung einer physikalischen Mischung der Komponenten unterstützt.

9. Implantatmaterial nach einem der Ansprüche 7 oder 8, **dadurch gekennzeichnet, dass** die Trägerflüssigkeit der ersten Komponente des Polymersystems und/oder die reaktive organische Flüssigkeit der zweiten Komponente des Polymersystems wasserlösliche Monomere oder Makromere enthalten, die während oder nach der Polymerisation des Polymersystems innerhalb der wässrigen Phase des sich ausbildenden polymerbasierten Feststoffs auspolymerisieren können,
wobei die wasserlöslichen Monomere ausgewählt sind aus Methacrylsäure, HEMA, HPMA, HEMA-Phosphat, Sulfopropyl-Methacrylat, deren Homologen oder deren Mischungen und/oder wobei die wasserlöslichen Makromere ausgewählt sind aus PEG-mono-Methacrylat, PEG-di-Methacrylat, verzweigten PEG-n-Methacrylaten, deren Homologen oder deren Mischungen.

10. Implantatmaterial nach einem der Ansprüche 1 bis 9, **dadurch gekennzeichnet, dass** die Komponenten des Polymersystems gelöste Polymere oder feinteilig suspendierte organische, anorganische oder organomineralische Bestandteile zur Einstellung der Viskosität der Komponenten des Polymersystems enthalten und dass die Viskosität der Komponenten des Polymersystems den Wert von 200 Pa*s nicht übersteigt.

11. Implantatmaterial nach einem der Ansprüche 1 bis 10, wobei mindestens eine der Komponenten des Polymersystems mikrokristalline, nanokristalline oder amorphe Knochenmineralien bzw. synthetische Knochen-analoge Mineralien enthält.

12. Implantatmaterial nach einem der Ansprüche 1 bis 11, **dadurch gekennzeichnet, dass** mindestens eine Komponente des Polymersystems mindestens eine bioverträgliche Substanz enthält, die als Kristallisationskeim für Mineralablagerungen dient und/oder die Ausbildung solcher Mineralablagerungen fördert und dass diese Substanzen mindestens eine Carboxyl-, Sulfat- und/oder Phosphatgruppe und/oder eine Siloxangruppe enthalten.

13. Applikationsset umfassend ein Innplantatmaterial nach einem der Ansprüche 1 bis 12 mit einer Doppelkammerspritze und einen Zwangsmischer/Statikmischer.

14. Verwendung eines Implantatmaterials nach einem der Ansprüche 1 bis 12 zur Herstellung von Knochenzementen, Knochenersatzmaterialien, Knochenklebern und/oder implantierbaren Wirkstoffträgern.

## Claims

1. Implant material based on a polymer system of at least two components which when mixed react with each other forming a polymer-based solid, with the first component of the polymer system containing at least one biocompatible polymer powder and an starter component for initiating a polymerization reaction during mixing and with the second component of the polymer system containing at least one reactive organic liquid or a solution or a suspension of a reactive organic liquid and a biocompatible polymer, **characterized by** that the first component of the polymer system is a storage stable paste that contains the polymer powder and the initiator component combined with a carrier liquid, wherein under normal conditions the polymer powder and the initiator component do not dissolve in the carrier liquid but are suspended therein and the initiator component remains stable until mixing with the second component of the polymer system.

2. Implant material according to claim 1 **characterized by** that the biocompatible polymer component is selected from homopolymers or copolymers of acrylic-acid esters, methacrylic-acid esters, styrene derivates, vinyl derivates or mixtures thereof and/or that the carrier liquid is selected from water, aqueous solutions, glycerol, propandiol, low-molecular PEG, PEG-PPG-copolymers, DMSO, methylpyrrolidone, biocompatible oils or mixtures thereof with each other or mixtures thereof with other substances.

3. Implant material according to any of the claims 1 or 2 **characterized by** that the second component contains methylmethacrylate or homologous esters of the methacrylic-acid or mixtures thereof as reactive organic liquid.

4. Implant material according to any of the claims 1 to 3 **characterized by** that the polymer of the second component is a polyacrylic-acid ester or a polymethacrylic-acid ester or a polystyrene or a copolymer thereof.

5. Implant material according to any of the claims 1 to 4 **characterized by** that the starter system is a 2-component radical starter, wherein one component of the starter system is contained in undissolved form in the carrier liquid in the first component of the polymer system and the second component of the starter system is contained in the second component of the polymer system as co-starter, polymerization accelerator or initiating agent.

6. Implant material according to any of the claims 1 to 5 **characterized by** that the first or the second or both components of the polymer system contain water-soluble polymers and/or antibiotics.

7. Implant material according to any of the claims 1 to 6 **characterized by** that the carrier liquid of the first component of the polymer system and the reactive organic liquid of the second component of the polymer system are chemically immiscible with each other or not or only poorly soluble in each other and that when these components are mixed a physical mixture is formed.

8. Implant material according to claim 7 **characterized by** that at least one component of the polymer system contains at least one anionic and/or non-ionic biocompatible tenside that supports the formation of a physical mixture of the components.

9. Implant material according to any of the claims 7 or 8 **characterized by** that the carrier liquid of the first component of the polymer system and/or the reactive organic liquid of the second component of the polymer system contain water-soluble monomers or macromers that during or after the polymerization of the polymer system can thorougly polymerize within the aqueous phase of the forming polymer-based solid, wherein the water-soluble monomers are selected from methacrylic acid, HEMA, HPMA, HEMA-phosphate, sulfopropyl methacrylate, homologs thereof or the mixtures thereof and/or wherein the water-soluble macromers are selected from PEG-mono-methacrylate, PEG-di-methacrylate, branched PEG-n-methacrylates, homologs thereof or mixtures thereof.

10. Implant material according to any of the claims 1 to 9 **characterized by** that the components of the polymer system contain dissolved polymers or organic, inorganic or organomineral constituents suspended in fine dispersion for setting the viscosity of the components of the polymer system and that the viscosity of the components of the polymer system does not exceed the value of 200 Pa*s.

11. Implant material according to any of the claims 1 to 10, wherein at least one of the components of the polymer system contains microcrystalline, nanocrystalline or amorphous bone minerals or synthetic bone-analogous minerals.

12. Implant material according to any of the claims 1 to 11 **characterized by** that at least one component of the polymer system contains at least one biocompatible substance that serves as crystallization seed for mineral deposits and/or promotes the formation of such mineral deposits and that these substances contain at least one carboxyl, sulfate and/or phosphate group and/or one siloxane group.

13. Application set comprising an implant material according to any of the claims 1 to 12 with a two-compartment syringe and a compulsory mixer/static mixer.

14. Use of an implant material according to any of the claims 1 to 12 for producing bone cements, bone replacement materials, bone adhesives and/or implantable carriers for active substances.

## Revendications

1. Matériau pour implant à base d'un système de polymères composé d'au moins 2 composants qui réagissent entre eux lors du mélange et forment une matière solide à base de polymères, le premier composant du système de polymères contenant au moins une poudre de polymère biocompatible et un composant d'amorçage pour déclencher une réaction de polymérisation lors du mélange et le deuxième composant du système de polymères contenant au moins un liquide organique réactif ou une solution ou une suspension d'un liquide organique réactif et d'un polymère biocompatible, **caractérisé en ce que** le premier composant du système de polymères est une pâte stable au stockage qui contient la poudre de polymère et le composant d'amorçage en combinaison avec un liquide porteur, la poudre de polymère et le composant d'amorçage ne se dissolvant pas dans le liquide porteur dans des conditions normales, mais étant présents en suspension et le composant d'amorçage restant stable jusqu'au mélange avec le deuxième composant du système de polymères.

2. Matériau pour implant selon la revendication 1, **caractérisé en ce que** le composant polymère biocompatible est sélectionné parmi les composés suivants : homo- ou copolymères d'esters d'acide acrylique, méthacrylique, de dérivés de styrène, de vinyle ou leur mélange et/ou que le liquide porteur est sélectionné parmi les composés suivants : eau, solutions aqueuses, glycérine, propanediol, PEG à faible poids moléculaire, copolymères PEG-PPG, DMSO, méthylpyrrolidone, huiles biocompatibles ou leurs mélanges entre eux ou leurs mélanges entre eux avec d'autres substances.

3. Matériau pour implant selon une des revendications 1 ou 2, **caractérisé en ce que** le deuxième composant contient du méthacrylate de méthyle ou des esters homologues de l'acide méthacrylique ou leurs mélanges en tant que liquide organique réactif.

4. Matériau pour implant selon une des revendications 1 à 3, **caractérisé en ce que** le polymère du deuxième composant est un ester d'acide polyacrylique ou un ester d'acide polyméthacrylique ou un polystyrène ou leurs copolymères.

5. Matériau pour implant selon une des revendications 1 à 4, **caractérisé en ce que** le système d'amorçage est un amorceur radicalaire à 2 composants, un composant du système d'amorçage étant contenu sous forme non dissoute dans le liquide porteur dans le premier composant du système de polymères et le deuxième composant du système d'amorçage en tant que co-amorceur, accélérateur de polymérisation ou initiateur dans le deuxième composant du système de polymères.

6. Matériau pour implant selon une des revendications 1 à 5, **caractérisé en ce que** le premier, le deuxième ou les deux composants du système de polymères contiennent des polymères hydrosolubles et/ou des antibiotiques.

7. Matériau pour implant selon une des revendications 1 à 6, **caractérisé en ce que** le liquide porteur du premier composant du système de polymères et le liquide organique réactif du deuxième composant du système de polymères ne sont pas miscibles entre eux au sens chimique, ou pas ou seulement faiblement solubles l'un dans l'autre, et qu'il apparaît un mélange physique lors du mélange de ces composants.

8. Matériau pour implant selon la revendication 7, **caractérisé en ce qu'**au moins un composant du système de polymères contient au moins un agent tensio-actif anionique et/ou non ionique biocompatible qui soutient la formation d'un mélange physique des composants.

9. Matériau pour implant selon une des revendications 7 ou 8, **caractérisé en ce que** le liquide porteur du premier composant du système de polymères et/ou le liquide organique réactif du deuxième composant du système de polymères contiennent des monomères ou des macromères hydrosolubles qui peuvent polymériser pendant ou après la polymérisation du système de polymères à l'intérieur de la phase aqueuse de la matière solide à base de polymères qui se forme,
les monomères hydrosolubles étant sélectionnés parmi les composés suivants : acide méthacrylique, HEMA, HPMA, phosphate de HEMA, méthacrylate de sulfopropyle, leurs homologues ou leurs mélanges et/ou les macromères hydrosolubles étant sélectionnés parmi les composés suivants : monométhacrylate de PEG, diméthacrylate de PEG, n-méthacrylates de PEG ramifiés, leurs homologues ou leurs mélanges.

10. Matériau pour implant selon une des revendications 1 à 9, **caractérisé en ce que** les composants du système de polymères contiennent des polymères dissous ou des constituants organiques, anorganiques ou organo-minéraux suspendus en fines particules pour régler la viscosité des composants du système de polymères et que la viscosité des composants du système de polymères ne dépasse pas la valeur de 200 Pa*s.

11. Matériau pour implant selon une des revendications 1 à 10, dans lequel au moins un des composants du système de polymères contient des minéraux osseux ou des minéraux synthétiques analogues aux minéraux osseux microcristallins, nanocristallins ou amorphes.

12. Matériau pour implant selon une des revendications 1 à 11, **caractérisé en ce qu'**au moins un composant du système de polymères contient au moins une substance biocompatible qui sert de germe de cristallisation pour les dépôts de minéraux et/ou favorise la formation de tels dépôts de minéraux et que ces substances contiennent au moins un groupe carboxyle, sulfate et/ou phosphate et/ou un groupe siloxane.

13. Kit d'application comprenant un matériau pour implant selon une des revendications 1 à 12 avec une seringue à double chambre et un mélangeur à mélange forcé/mélangeur statique.

14. Utilisation d'un matériau pour implant selon une des revendications 1 à 12 pour la fabrication d'éléments osseux, de matériaux de substitution osseuse, de colles osseuses et/ou de supports de principe actif implantables.
